# EUROPEAN PATENT APPLICATION

(11) **EP 3 995 578 A1**
(43) Date of publication of application: **11.05.2022**
(21) Application number: 20834537.1
(22) Date of filing: 03.07.2020
(51) Int. Cl.: C12N 15/113, C12N 15/63, A61K 31/7088, A61K 48/00, A61P 27/16

(54) **OLIGOMERIC NUCLEIC ACID MOLECULE ACTIVATING ATOH1 GENE AND USE THEREOF**

(30) Priority: 04.07.2019 CN 201910598221
(71) Applicant: Ractigen Therapeutics, Nantong, Jiangsu 226400 (CN)
(72) Inventor: LI, Longcheng, Nantong, Jiangsu 226400 (CN); KANG, Moorim, Nantong, Jiangsu 226400 (CN)
(74) Representative: Høiberg P/S
(86) International application number: PCT/CN2020/100093
(87) International publication number: WO 2021/000928

(57) **Abstract**

The present invention relates to oligomeric nucleic acid molecules and uses thereof for treating hearing loss. The present invention relates to small activating nucleic acid molecules for treating hearing loss. The small activating nucleic acid molecules of the present invention can be double-stranded or single-stranded RNA molecules targeting the promoter region of an *ATOH1* gene comprising a first nucleic acid strand and a second nucleic acid strand. The double-stranded RNA molecule targeting the promoter region of the *ATOH1* gene comprises two nucleic acid strands of 16 to 35 nucleotides in length, wherein one nucleic acid strand has at least 75% homology or complementarity to a target selected from the promoter region of the *ATOH1* gene. The present invention also relates to pharmaceutical compositions comprising the small activating nucleic acid molecules and optionally, a pharmaceutically acceptable carrier, and methods for upregulating the expression of the *ATOH1* gene and ATOH1 protein in a cell and treating diseases or conditions, related to insufficient or decreased expression of the *ATOH1* gene by using the small activating nucleic acid molecules, or the pharmaceutical composition.

SEQUENCE LISTING

<110>  Ractigen Therapeutics

<120>  OLIGOMERIC NUCLEIC ACID MOLECULE FOR ACTIVATING ATOH1 GENE AND
       USE THEREOF

<130>  065786.11049/7US1

<150>  CN 201910598221.6
<151>  2019-07-04

<150>  PCT/CN2020/100093
<151>  2020-07-03

<160>  424

<170>  PatentIn version 3.5

<210>  1
<211>  637
<212>  DNA
<213>  Homo sapiens

<400>  1
aaatgcttgg gcatcagaag tgggagctgt gatcctagct tgggggcagc acagggtagg      60

cggccttctc tctgctttga gtggcttctg ggcgcctggc gggtccagaa tcgcccagag     120

ccgcccgcgg tcgtgcacat ctgacccgag tcagcttggg caccagccga gagccggctc     180

cgcaccgctc ccgcacccca gccgccgggg tggtgacaca caccggagtc gaattacagc     240

cctgcaatta acatatgaat ctgacgaatt taaaagaagg aaaaaaaaaa aaaaacctga     300

gcaggcttgg gagtcctctg cacacaagaa cttttctcgg ggtgtaaaaa ctctttgatt     360

ggctgctcgc acgcgcctgc ccgcgccctc cattggctga gaagacacgc gaccggcgcg     420

aggagggggt tgggagagga gcggggggag actgagtggc gcgtgccgct ttttaaaggg     480

gcgcagcgcc ttcagcaacc ggagaagcat agttgcacgc gacctggtgt gtgatctccg     540

agtgggtggg ggagggtcga ggagggaaaa aaaaataaga cgttgcagaa gagacccgga     600

aagggccttt tttttggttg agctggtgtc ccagtgc                              637


<210>  2
<211>  192
<212>  DNA
<213>  Homo sapiens

<400>  2
gcggccttct ctctgctttg agtggcttct gggcgcctgg cgggtccaga atcgcccaga      60

gccgcccgcg gtcgtgcaca tctgacccga gtcagcttgg gcaccagccg agagccggct     120

ccgcaccgct cccgcacccc agccgccggg gtggtgacac acaccggagt cgaattacag     180

ccctgcaatt aa                                                         192

<210>  3
<211>  29
<212>  DNA
<213>  Homo sapiens

<400>  3
tgcacacaag aacttttctc ggggtgtaa                                          29


<210>  4
<211>  76
<212>  DNA
<213>  Homo sapiens

<400>  4
aaggggcgca gcgccttcag caaccggaga agcatagttg cacgcgacct ggtgtgtgat        60

ctccgagtgg gtgggg                                                        76


<210>  5
<211>  22
<212>  DNA
<213>  Homo sapiens

<400>  5
ttgcagaaga gacccggaaa gg                                                 22


<210>  6
<211>  21
<212>  DNA
<213>  Artificial Sequence

<220>
<223>  Synthetic DNA/RNA construct

<400>  6
gcggccuucu cucugcuuut t                                                  21


<210>  7
<211>  21
<212>  DNA
<213>  Artificial Sequence

<220>
<223>  Synthetic DNA/RNA construct

<400>  7
cagaagagac ccggaaaggt t                                                  21


<210>  8
<211>  21
<212>  DNA
<213>  Artificial Sequence

<220>
<223>  Synthetic DNA/RNA construct

<400>  8
ccggagaagc auaguugcat t                                                  21

<210>  9
<211>  21
<212>  DNA
<213>  Artificial Sequence

<220>
<223>  Synthetic DNA/RNA construct

<400>  9
cgcgaccugg ugugugauct t                                                21


<210>  10
<211>  21
<212>  DNA
<213>  Artificial Sequence

<220>
<223>  Synthetic DNA/RNA construct

<400>  10
gagcaggcuu gggaguccut t                                                21


<210>  11
<211>  21
<212>  DNA
<213>  Artificial Sequence

<220>
<223>  Synthetic DNA/RNA construct

<400>  11
uaguugcacg cgaccuggut t                                                21


<210>  12
<211>  21
<212>  DNA
<213>  Artificial Sequence

<220>
<223>  Synthetic DNA/RNA construct

<400>  12
gguccagaau cgcccagagt t                                                21


<210>  13
<211>  21
<212>  DNA
<213>  Artificial Sequence

<220>
<223>  Synthetic DNA/RNA construct

<400>  13
gcagaagaga cccggaaagt t                                                21


<210>  14

<211>  21
<212>  DNA
<213>  Artificial Sequence

<220>
<223>  Synthetic DNA/RNA construct

<400>  14
ccgcggucgu gcacaucugt t                                                21


<210>  15
<211>  21
<212>  DNA
<213>  Artificial Sequence

<220>
<223>  Synthetic DNA/RNA construct

<400>  15
cggguccaga aucgcccagt t                                                21


<210>  16
<211>  21
<212>  DNA
<213>  Artificial Sequence

<220>
<223>  Synthetic DNA/RNA construct

<400>  16
gccuucagca accggagaat t                                                21


<210>  17
<211>  21
<212>  DNA
<213>  Artificial Sequence

<220>
<223>  Synthetic DNA/RNA construct

<400>  17
acaccggagu cgaauuacat t                                                21


<210>  18
<211>  21
<212>  DNA
<213>  Artificial Sequence

<220>
<223>  Synthetic DNA/RNA construct

<400>  18
aacuuuucuc gggguguaat t                                                21


<210>  19
<211>  21
<212>  DNA
<213>  Artificial Sequence

<220>
<223>  Synthetic DNA/RNA construct

<400>  19
uugggagucc ucugcacact t                                                21


<210>  20
<211>  21
<212>  DNA
<213>  Artificial Sequence

<220>
<223>  Synthetic DNA/RNA construct

<400>  20
uugggcacca gccgagagct t                                                21


<210>  21
<211>  21
<212>  DNA
<213>  Artificial Sequence

<220>
<223>  Synthetic DNA/RNA construct

<400>  21
ugcagaagag acccggaaat t                                                21


<210>  22
<211>  21
<212>  DNA
<213>  Artificial Sequence

<220>
<223>  Synthetic DNA/RNA construct

<400>  22
ccagaaucgc ccagagccgt t                                                21


<210>  23
<211>  21
<212>  DNA
<213>  Artificial Sequence

<220>
<223>  Synthetic DNA/RNA construct

<400>  23
ggcgcagcgc cuucagcaat t                                                21


<210>  24
<211>  21
<212>  DNA
<213>  Artificial Sequence

<220>
<223>  Synthetic DNA/RNA construct

<400>  24
cacgcgaccu ggugugugat t                                                21


<210>  25
<211>  21
<212>  DNA
<213>  Artificial Sequence

<220>
<223>  Synthetic DNA/RNA construct

<400>  25
gcggguccag aaucgcccat t                                                21


<210>  26
<211>  21
<212>  DNA
<213>  Artificial Sequence

<220>
<223>  Synthetic DNA/RNA construct

<400>  26
uucucucugc uuugaguggt t                                                21


<210>  27
<211>  21
<212>  DNA
<213>  Artificial Sequence

<220>
<223>  Synthetic DNA/RNA construct

<400>  27
agccgagagc cggcuccgct t                                                21


<210>  28
<211>  21
<212>  DNA
<213>  Artificial Sequence

<220>
<223>  Synthetic DNA/RNA construct

<400>  28
cacaucugac ccgagucagt t                                                21


<210>  29
<211>  21
<212>  DNA
<213>  Artificial Sequence

<220>
<223>  Synthetic DNA/RNA construct

<400>  29
gccgagagcc ggcuccgcat t                                                21

<210>  30
<211>  21
<212>  DNA
<213>  Artificial Sequence

<220>
<223>  Synthetic DNA/RNA construct

<400>  30
gcuugggcac cagccgagat t                                                21


<210>  31
<211>  21
<212>  DNA
<213>  Artificial Sequence

<220>
<223>  Synthetic DNA/RNA construct

<400>  31
uugauuggcu gcucgcacgt t                                                21


<210>  32
<211>  21
<212>  DNA
<213>  Artificial Sequence

<220>
<223>  Synthetic DNA/RNA construct

<400>  32
gccgggugg ugacacacat t                                                 21


<210>  33
<211>  21
<212>  DNA
<213>  Artificial Sequence

<220>
<223>  Synthetic DNA/RNA construct

<400>  33
ccuccauugg cugagaagat t                                                21


<210>  34
<211>  21
<212>  DNA
<213>  Artificial Sequence

<220>
<223>  Synthetic DNA/RNA construct

<400>  34
uucagcaacc ggagaagcat t                                                21


<210>  35

<211>  21
<212>  DNA
<213>  Artificial Sequence

<220>
<223>  Synthetic DNA/RNA construct

<400>  35
ccuucagcaa ccggagaagt t                                                21


<210>  36
<211>  21
<212>  DNA
<213>  Artificial Sequence

<220>
<223>  Synthetic DNA/RNA construct

<400>  36
gcaggcuugg gaguccucut t                                                21


<210>  37
<211>  21
<212>  DNA
<213>  Artificial Sequence

<220>
<223>  Synthetic DNA/RNA construct

<400>  37
agcaggcuug ggaguccuct t                                                21


<210>  38
<211>  21
<212>  DNA
<213>  Artificial Sequence

<220>
<223>  Synthetic DNA/RNA construct

<400>  38
agcuugggca ccagccgagt t                                                21


<210>  39
<211>  21
<212>  DNA
<213>  Artificial Sequence

<220>
<223>  Synthetic DNA/RNA construct

<400>  39
caggcuuggg aguccucugt t                                                21


<210>  40
<211>  21
<212>  DNA
<213>  Artificial Sequence

<220>
<223>  Synthetic DNA/RNA construct

<400>  40
gcacaucuga cccgagucat t                                                21


<210>  41
<211>  21
<212>  DNA
<213>  Artificial Sequence

<220>
<223>  Synthetic DNA/RNA construct

<400>  41
gcgccuucag caaccggagt t                                                21


<210>  42
<211>  21
<212>  DNA
<213>  Artificial Sequence

<220>
<223>  Synthetic DNA/RNA construct

<400>  42
accuggugug ugaucuccgt t                                                21


<210>  43
<211>  21
<212>  DNA
<213>  Artificial Sequence

<220>
<223>  Synthetic DNA/RNA construct

<400>  43
cgaauuacag cccugcaaut t                                                21


<210>  44
<211>  21
<212>  DNA
<213>  Artificial Sequence

<220>
<223>  Synthetic DNA/RNA construct

<400>  44
ucgaauuaca gcccugcaat t                                                21


<210>  45
<211>  21
<212>  DNA
<213>  Artificial Sequence

<220>
<223>  Synthetic DNA/RNA construct

<400>  45
cacacaagaa cuuuucucgt t                                                21

<210>  46
<211>  21
<212>  DNA
<213>  Artificial Sequence

<220>
<223>  Synthetic DNA/RNA construct

<400>  46
ugggaguccu cugcacacat t                                                21

<210>  47
<211>  21
<212>  DNA
<213>  Artificial Sequence

<220>
<223>  Synthetic DNA/RNA construct

<400>  47
gggagcugug auccuagcut t                                                21

<210>  48
<211>  21
<212>  DNA
<213>  Artificial Sequence

<220>
<223>  Synthetic DNA/RNA construct

<400>  48
cgccuucagc aaccggagat t                                                21

<210>  49
<211>  21
<212>  DNA
<213>  Artificial Sequence

<220>
<223>  Synthetic DNA/RNA construct

<400>  49
ggagcuguga uccuagcuut t                                                21

<210>  50
<211>  21
<212>  DNA
<213>  Artificial Sequence

<220>
<223>  Synthetic DNA/RNA construct

<400>  50
gagucgaauu acagcccugt t                                                21

<210>  51
<211>  21
<212>  DNA
<213>  Artificial Sequence

<220>
<223>  Synthetic DNA/RNA construct

<400>  51
aaggggcgca gcgccuucat t                                                21


<210>  52
<211>  21
<212>  DNA
<213>  Artificial Sequence

<220>
<223>  Synthetic DNA/RNA construct

<400>  52
caccggaguc gaauuacagt t                                                21


<210>  53
<211>  21
<212>  DNA
<213>  Artificial Sequence

<220>
<223>  Synthetic DNA/RNA construct

<400>  53
uccucugcac acaagaacut t                                                21


<210>  54
<211>  21
<212>  DNA
<213>  Artificial Sequence

<220>
<223>  Synthetic DNA/RNA construct

<400>  54
caccgcuccc gcaccccagt t                                                21


<210>  55
<211>  21
<212>  DNA
<213>  Artificial Sequence

<220>
<223>  Synthetic DNA/RNA construct

<400>  55
guccucugca cacaagaact t                                                21


<210>  56

<211>  21
<212>  DNA
<213>  Artificial Sequence

<220>
<223>  Synthetic DNA/RNA construct

<400>  56
acaucugacc cgagucagct t                                                21


<210>  57
<211>  21
<212>  DNA
<213>  Artificial Sequence

<220>
<223>  Synthetic DNA/RNA construct

<400>  57
cggccuucuc ucugcuuugt t                                                21


<210>  58
<211>  21
<212>  DNA
<213>  Artificial Sequence

<220>
<223>  Synthetic DNA/RNA construct

<400>  58
ggagacugag uggcgcgugt t                                                21


<210>  59
<211>  21
<212>  DNA
<213>  Artificial Sequence

<220>
<223>  Synthetic DNA/RNA construct

<400>  59
cuucagcaac cggagaagct t                                                21


<210>  60
<211>  21
<212>  DNA
<213>  Artificial Sequence

<220>
<223>  Synthetic DNA/RNA construct

<400>  60
aucgcccaga gccgcccgct t                                                21


<210>  61
<211>  21
<212>  DNA
<213>  Artificial Sequence

<220>
<223>  Synthetic DNA/RNA construct

<400>  61
uucugggcgc cuggcgggut t                                                21


<210>  62
<211>  21
<212>  DNA
<213>  Artificial Sequence

<220>
<223>  Synthetic DNA/RNA construct

<400>  62
uuggcugcuc gcacgcgcct t                                                21


<210>  63
<211>  21
<212>  DNA
<213>  Artificial Sequence

<220>
<223>  Synthetic DNA/RNA construct

<400>  63
cuugggcacc agccgagagt t                                                21


<210>  64
<211>  21
<212>  DNA
<213>  Artificial Sequence

<220>
<223>  Synthetic DNA/RNA construct

<400>  64
ggcaucagaa gugggagcut t                                                21


<210>  65
<211>  21
<212>  DNA
<213>  Artificial Sequence

<220>
<223>  Synthetic DNA/RNA construct

<400>  65
ggcgggucca gaaucgccct t                                                21


<210>  66
<211>  21
<212>  DNA
<213>  Artificial Sequence

<220>
<223>  Synthetic DNA/RNA construct

<400>  66
caucagaagu gggagcugut t                                                21


<210>  67
<211>  21
<212>  DNA
<213>  Artificial Sequence

<220>
<223>  Synthetic DNA/RNA construct

<400>  67
gcccgcgguc gugcacauct t                                                21


<210>  68
<211>  21
<212>  DNA
<213>  Artificial Sequence

<220>
<223>  Synthetic DNA/RNA construct

<400>  68
uuacagcccu gcaauuaact t                                                21


<210>  69
<211>  21
<212>  DNA
<213>  Artificial Sequence

<220>
<223>  Synthetic DNA/RNA construct

<400>  69
uugcagaaga gacccggaat t                                                21


<210>  70
<211>  21
<212>  DNA
<213>  Artificial Sequence

<220>
<223>  Synthetic DNA/RNA construct

<400>  70
aggggcgcag cgccuucagt t                                                21


<210>  71
<211>  21
<212>  DNA
<213>  Artificial Sequence

<220>
<223>  Synthetic DNA/RNA construct

<400>  71
cucugcacac aagaacuuut t                                                21

<210>  72
<211>  21
<212>  DNA
<213>  Artificial Sequence

<220>
<223>  Synthetic DNA/RNA construct

<400>  72
ugaucuccga gugggugggt t                                                21


<210>  73
<211>  21
<212>  DNA
<213>  Artificial Sequence

<220>
<223>  Synthetic DNA/RNA construct

<400>  73
guugcagaag agacccggat t                                                21


<210>  74
<211>  21
<212>  DNA
<213>  Artificial Sequence

<220>
<223>  Synthetic DNA/RNA construct

<400>  74
cugcacacaa gaacuuuuct t                                                21


<210>  75
<211>  21
<212>  DNA
<213>  Artificial Sequence

<220>
<223>  Synthetic DNA/RNA construct

<400>  75
cgagucagcu ugggcaccat t                                                21


<210>  76
<211>  21
<212>  DNA
<213>  Artificial Sequence

<220>
<223>  Synthetic DNA/RNA construct

<400>  76
gucagcuugg gcaccagcct t                                                21


<210>  77

<211>  21
<212>  DNA
<213>  Artificial Sequence

<220>
<223>  Synthetic DNA/RNA construct

<400>  77
ggagucgaau uacagcccut t                                                21


<210>  78
<211>  21
<212>  DNA
<213>  Artificial Sequence

<220>
<223>  Synthetic DNA/RNA construct

<400>  78
cgcggucgug cacaucugat t                                                21


<210>  79
<211>  21
<212>  DNA
<213>  Artificial Sequence

<220>
<223>  Synthetic DNA/RNA construct

<400>  79
ugguguguga ucuccgagut t                                                21


<210>  80
<211>  21
<212>  DNA
<213>  Artificial Sequence

<220>
<223>  Synthetic DNA/RNA construct

<400>  80
ugcucgcacg cgccugccct t                                                21


<210>  81
<211>  21
<212>  DNA
<213>  Artificial Sequence

<220>
<223>  Synthetic DNA/RNA construct

<400>  81
ugcuugggca ucagaagugt t                                                21


<210>  82
<211>  21
<212>  DNA
<213>  Artificial Sequence

<220>
<223>  Synthetic DNA/RNA construct

<400>  82
aguggcuucu gggcgccugt t                                                21


<210>  83
<211>  21
<212>  DNA
<213>  Artificial Sequence

<220>
<223>  Synthetic DNA/RNA construct

<400>  83
aguccucugc acacaagaat t                                                21


<210>  84
<211>  21
<212>  DNA
<213>  Artificial Sequence

<220>
<223>  Synthetic DNA/RNA construct

<400>  84
ugugugaucu ccgagugggt t                                                21


<210>  85
<211>  21
<212>  DNA
<213>  Artificial Sequence

<220>
<223>  Synthetic DNA/RNA construct

<400>  85
ugagcaggcu ugggagucct t                                                21


<210>  86
<211>  21
<212>  DNA
<213>  Artificial Sequence

<220>
<223>  Synthetic DNA/RNA construct

<400>  86
cgugcacauc ugacccgagt t                                                21


<210>  87
<211>  21
<212>  DNA
<213>  Artificial Sequence

<220>
<223>  Synthetic DNA/RNA construct

<400>  87
ucgugcacau cugacccgat t                                                21


<210>  88
<211>  21
<212>  DNA
<213>  Artificial Sequence

<220>
<223>  Synthetic DNA/RNA construct

<400>  88
agcuucugau uggcucuuut t                                                21


<210>  89
<211>  21
<212>  DNA
<213>  Artificial Sequence

<220>
<223>  Synthetic DNA/RNA construct

<400>  89
gucgugcaca ucugacccgt t                                                21


<210>  90
<211>  21
<212>  DNA
<213>  Artificial Sequence

<220>
<223>  Synthetic DNA/RNA construct

<400>  90
cacaggguag gcggccuuct t                                                21


<210>  91
<211>  21
<212>  DNA
<213>  Artificial Sequence

<220>
<223>  Synthetic DNA/RNA construct

<400>  91
cuggugugug aucuccgagt t                                                21


<210>  92
<211>  21
<212>  DNA
<213>  Artificial Sequence

<220>
<223>  Synthetic DNA/RNA construct

<400>  92
gcaccgcucc cgcaccccat t                                                21

<210>  93
<211>  21
<212>  DNA
<213>  Artificial Sequence

<220>
<223>  Synthetic DNA/RNA construct

<400>  93
aagaacuuuu cucggggugt t                                                21


<210>  94
<211>  21
<212>  DNA
<213>  Artificial Sequence

<220>
<223>  Synthetic DNA/RNA construct

<400>  94
cacaagaacu uuucucgggt t                                                21


<210>  95
<211>  21
<212>  DNA
<213>  Artificial Sequence

<220>
<223>  Synthetic DNA/RNA construct

<400>  95
gccuucucuc ugcuuugagt t                                                21


<210>  96
<211>  21
<212>  DNA
<213>  Artificial Sequence

<220>
<223>  Synthetic DNA/RNA construct

<400>  96
gagccggcuc cgcaccgcut t                                                21


<210>  97
<211>  21
<212>  DNA
<213>  Artificial Sequence

<220>
<223>  Synthetic DNA/RNA construct

<400>  97
acgcgaccgg cgcgaggagt t                                                21


<210>  98

<211>  21
<212>  DNA
<213>  Artificial Sequence

<220>
<223>  Synthetic DNA/RNA construct

<400>  98
aaaugcuugg gcaucagaat t                                                21


<210>  99
<211>  21
<212>  DNA
<213>  Artificial Sequence

<220>
<223>  Synthetic DNA/RNA construct

<400>  99
gcuugggcau cagaaguggt t                                                21


<210>  100
<211>  21
<212>  DNA
<213>  Artificial Sequence

<220>
<223>  Synthetic DNA/RNA construct

<400>  100
agccgcccgc ggucgugcat t                                                21


<210>  101
<211>  21
<212>  DNA
<213>  Artificial Sequence

<220>
<223>  Synthetic DNA/RNA construct

<400>  101
ggucgugcac aucugaccct t                                                21


<210>  102
<211>  21
<212>  DNA
<213>  Artificial Sequence

<220>
<223>  Synthetic DNA/RNA construct

<400>  102
ucagcaaccg gagaagcaut t                                                21


<210>  103
<211>  21
<212>  DNA
<213>  Artificial Sequence

<220>
<223>  Synthetic DNA/RNA construct

<400>  103
agaaucgccc agagccgcct t                                                21


<210>  104
<211>  21
<212>  DNA
<213>  Artificial Sequence

<220>
<223>  Synthetic DNA/RNA construct

<400>  104
gagcugugau ccuagcuugt t                                                21


<210>  105
<211>  21
<212>  DNA
<213>  Artificial Sequence

<220>
<223>  Synthetic DNA/RNA construct

<400>  105
cggagaagca uaguugcact t                                                21


<210>  106
<211>  21
<212>  DNA
<213>  Artificial Sequence

<220>
<223>  Synthetic DNA/RNA construct

<400>  106
auaguugcac gcgaccuggt t                                                21


<210>  107
<211>  21
<212>  DNA
<213>  Artificial Sequence

<220>
<223>  Synthetic DNA/RNA construct

<400>  107
acuuuucucg ggguguaaat t                                                21


<210>  108
<211>  21
<212>  DNA
<213>  Artificial Sequence

<220>
<223>  Synthetic DNA/RNA construct

<400>  108
cuucugggcg ccuggcgggt t                                                21


<210>  109
<211>  21
<212>  DNA
<213>  Artificial Sequence

<220>
<223>  Synthetic DNA/RNA construct

<400>  109
cagccgccgg gguggugact t                                                21


<210>  110
<211>  21
<212>  DNA
<213>  Artificial Sequence

<220>
<223>  Synthetic DNA/RNA construct

<400>  110
agcugugauc cuagcuuggt t                                                21


<210>  111
<211>  21
<212>  DNA
<213>  Artificial Sequence

<220>
<223>  Synthetic DNA/RNA construct

<400>  111
gcgaccuggu gugugaucut t                                                21


<210>  112
<211>  21
<212>  DNA
<213>  Artificial Sequence

<220>
<223>  Synthetic DNA/RNA construct

<400>  112
agcacagggu aggcggccut t                                                21


<210>  113
<211>  21
<212>  DNA
<213>  Artificial Sequence

<220>
<223>  Synthetic DNA/RNA construct

<400>  113
gcgccuggcg gguccagaat t                                                21

<210>  114
<211>  21
<212>  DNA
<213>  Artificial Sequence

<220>
<223>  Synthetic DNA/RNA construct

<400>  114
gcaucagaag ugggagcugt t                                                21


<210>  115
<211>  21
<212>  DNA
<213>  Artificial Sequence

<220>
<223>  Synthetic DNA/RNA construct

<400>  115
gaaucgccca gagccgccct t                                                21


<210>  116
<211>  21
<212>  DNA
<213>  Artificial Sequence

<220>
<223>  Synthetic DNA/RNA construct

<400>  116
gggcgcagcg ccuucagcat t                                                21


<210>  117
<211>  21
<212>  DNA
<213>  Artificial Sequence

<220>
<223>  Synthetic DNA/RNA construct

<400>  117
uacagcccug caauuaacat t                                                21


<210>  118
<211>  21
<212>  DNA
<213>  Artificial Sequence

<220>
<223>  Synthetic DNA/RNA construct

<400>  118
aacauaugaa ucugacgaat t                                                21


<210>  119

<211>  21
<212>  DNA
<213>  Artificial Sequence

<220>
<223>  Synthetic DNA/RNA construct

<400>  119
accgcucccg caccccagct t                                                21


<210>  120
<211>  21
<212>  DNA
<213>  Artificial Sequence

<220>
<223>  Synthetic DNA/RNA construct

<400>  120
agacugagug gcgcgugcct t                                                21


<210>  121
<211>  21
<212>  DNA
<213>  Artificial Sequence

<220>
<223>  Synthetic DNA/RNA construct

<400>  121
gcauaguugc acgcgaccut t                                                21


<210>  122
<211>  21
<212>  DNA
<213>  Artificial Sequence

<220>
<223>  Synthetic DNA/RNA construct

<400>  122
ugcacaucug acccgaguct t                                                21


<210>  123
<211>  21
<212>  DNA
<213>  Artificial Sequence

<220>
<223>  Synthetic DNA/RNA construct

<400>  123
aaucgcccag agccgcccgt t                                                21


<210>  124
<211>  21
<212>  DNA
<213>  Artificial Sequence

<220>
<223>  Synthetic DNA/RNA construct

<400>  124
cccgagucag cuugggcact t                                                21


<210>  125
<211>  21
<212>  DNA
<213>  Artificial Sequence

<220>
<223>  Synthetic DNA/RNA construct

<400>  125
ugggcaccag ccgagagcct t                                                21


<210>  126
<211>  21
<212>  DNA
<213>  Artificial Sequence

<220>
<223>  Synthetic DNA/RNA construct

<400>  126
cuuuucucgg gguguaaaat t                                                21


<210>  127
<211>  21
<212>  DNA
<213>  Artificial Sequence

<220>
<223>  Synthetic DNA/RNA construct

<400>  127
gcuuugagug gcuucugggt t                                                21


<210>  128
<211>  21
<212>  DNA
<213>  Artificial Sequence

<220>
<223>  Synthetic DNA/RNA construct

<400>  128
gucgaauuac agcccugcat t                                                21


<210>  129
<211>  21
<212>  DNA
<213>  Artificial Sequence

<220>
<223>  Synthetic DNA/RNA construct

<400>  129
gcggucgugc acaucugact t                                                21


<210>  130
<211>  21
<212>  DNA
<213>  Artificial Sequence

<220>
<223>  Synthetic DNA/RNA construct

<400>  130
aauuacagcc cugcaauuat t                                                21


<210>  131
<211>  21
<212>  DNA
<213>  Artificial Sequence

<220>
<223>  Synthetic DNA/RNA construct

<400>  131
acguugcaga agagacccgt t                                                21


<210>  132
<211>  21
<212>  DNA
<213>  Artificial Sequence

<220>
<223>  Synthetic DNA/RNA construct

<400>  132
ggcgccuggc ggguccagat t                                                21


<210>  133
<211>  21
<212>  DNA
<213>  Artificial Sequence

<220>
<223>  Synthetic DNA/RNA construct

<400>  133
gcacgcgacc uggugugugt t                                                21


<210>  134
<211>  21
<212>  DNA
<213>  Artificial Sequence

<220>
<223>  Synthetic DNA/RNA construct

<400>  134
ugcacgcgac cuggugugut t                                                21

<210>  135
<211>  21
<212>  DNA
<213>  Artificial Sequence

<220>
<223>  Synthetic DNA/RNA construct

<400>  135
ccgccggggu ggugacacat t                                                21


<210>  136
<211>  21
<212>  DNA
<213>  Artificial Sequence

<220>
<223>  Synthetic DNA/RNA construct

<400>  136
ucuuugauug gcugcucgct t                                                21


<210>  137
<211>  21
<212>  DNA
<213>  Artificial Sequence

<220>
<223>  Synthetic DNA/RNA construct

<400>  137
acacaccgga gucgaauuat t                                                21


<210>  138
<211>  21
<212>  DNA
<213>  Artificial Sequence

<220>
<223>  Synthetic DNA/RNA construct

<400>  138
aaugcuuggg caucagaagt t                                                21


<210>  139
<211>  21
<212>  DNA
<213>  Artificial Sequence

<220>
<223>  Synthetic DNA/RNA construct

<400>  139
aacucuuuga uuggcugcut t                                                21


<210>  140

<211>  21
<212>  DNA
<213>  Artificial Sequence

<220>
<223>  Synthetic DNA/RNA construct

<400>  140
ggguccagaa ucgcccagat t                                                21


<210>  141
<211>  21
<212>  DNA
<213>  Artificial Sequence

<220>
<223>  Synthetic DNA/RNA construct

<400>  141
cacaccggag ucgaauuact t                                                21


<210>  142
<211>  21
<212>  DNA
<213>  Artificial Sequence

<220>
<223>  Synthetic DNA/RNA construct

<400>  142
gaguccucug cacacaagat t                                                21


<210>  143
<211>  21
<212>  DNA
<213>  Artificial Sequence

<220>
<223>  Synthetic DNA/RNA construct

<400>  143
aaagcagaga gaaggccgct t                                                21


<210>  144
<211>  21
<212>  DNA
<213>  Artificial Sequence

<220>
<223>  Synthetic DNA/RNA construct

<400>  144
ccuuuccggg ucucuucugt t                                                21


<210>  145
<211>  21
<212>  DNA
<213>  Artificial Sequence

<220>
<223>  Synthetic DNA/RNA construct

<400>  145
ugcaacuaug cuucuccggt t                                                21


<210>  146
<211>  21
<212>  DNA
<213>  Artificial Sequence

<220>
<223>  Synthetic DNA/RNA construct

<400>  146
gaucacacac caggucgcgt t                                                21


<210>  147
<211>  21
<212>  DNA
<213>  Artificial Sequence

<220>
<223>  Synthetic DNA/RNA construct

<400>  147
aggacuccca agccugcuct t                                                21


<210>  148
<211>  21
<212>  DNA
<213>  Artificial Sequence

<220>
<223>  Synthetic DNA/RNA construct

<400>  148
accaggucgc gugcaacuat t                                                21


<210>  149
<211>  21
<212>  DNA
<213>  Artificial Sequence

<220>
<223>  Synthetic DNA/RNA construct

<400>  149
cucugggcga uucuggacct t                                                21


<210>  150
<211>  21
<212>  DNA
<213>  Artificial Sequence

<220>
<223>  Synthetic DNA/RNA construct

<400>  150
cuuuccgggu cucuucugct t                                                 21


<210>  151
<211>  21
<212>  DNA
<213>  Artificial Sequence

<220>
<223>  Synthetic DNA/RNA construct

<400>  151
cagaugugca cgaccgcggt t                                                 21


<210>  152
<211>  21
<212>  DNA
<213>  Artificial Sequence

<220>
<223>  Synthetic DNA/RNA construct

<400>  152
cugggcgauu cuggacccgt t                                                 21


<210>  153
<211>  21
<212>  DNA
<213>  Artificial Sequence

<220>
<223>  Synthetic DNA/RNA construct

<400>  153
uucuccgguu gcugaaggct t                                                 21


<210>  154
<211>  21
<212>  DNA
<213>  Artificial Sequence

<220>
<223>  Synthetic DNA/RNA construct

<400>  154
uguaauucga cuccggugut t                                                 21


<210>  155
<211>  21
<212>  DNA
<213>  Artificial Sequence

<220>
<223>  Synthetic DNA/RNA construct

<400>  155
uuacaccccg agaaaaguut t                                                 21

<210>  156
<211>  21
<212>  DNA
<213>  Artificial Sequence

<220>
<223>  Synthetic DNA/RNA construct

<400>  156
gugugcagag gacucccaat t                                                21


<210>  157
<211>  21
<212>  DNA
<213>  Artificial Sequence

<220>
<223>  Synthetic DNA/RNA construct

<400>  157
gcucucggcu ggugcccaat t                                                21


<210>  158
<211>  21
<212>  DNA
<213>  Artificial Sequence

<220>
<223>  Synthetic DNA/RNA construct

<400>  158
uuuccggguc ucuucugcat t                                                21


<210>  159
<211>  21
<212>  DNA
<213>  Artificial Sequence

<220>
<223>  Synthetic DNA/RNA construct

<400>  159
cggcucuggg cgauucuggt t                                                21


<210>  160
<211>  21
<212>  DNA
<213>  Artificial Sequence

<220>
<223>  Synthetic DNA/RNA construct

<400>  160
uugcugaagg cgcugcgcct t                                                21


<210>  161

<211>  21
<212>  DNA
<213>  Artificial Sequence

<220>
<223>  Synthetic DNA/RNA construct

<400>  161
ucacacacca ggucgcgugt t                                                21


<210>  162
<211>  21
<212>  DNA
<213>  Artificial Sequence

<220>
<223>  Synthetic DNA/RNA construct

<400>  162
ugggcgauuc uggacccgct t                                                21


<210>  163
<211>  21
<212>  DNA
<213>  Artificial Sequence

<220>
<223>  Synthetic DNA/RNA construct

<400>  163
ccacucaaag cagagagaat t                                                21


<210>  164
<211>  21
<212>  DNA
<213>  Artificial Sequence

<220>
<223>  Synthetic DNA/RNA construct

<400>  164
gcggagccgg cucucggcut t                                                21


<210>  165
<211>  21
<212>  DNA
<213>  Artificial Sequence

<220>
<223>  Synthetic DNA/RNA construct

<400>  165
cugacucggg ucagaugugt t                                                21


<210>  166
<211>  21
<212>  DNA
<213>  Artificial Sequence

<220>
<223>  Synthetic DNA/RNA construct

<400>  166
ugcggagccg gcucucggct t                                                21


<210>  167
<211>  21
<212>  DNA
<213>  Artificial Sequence

<220>
<223>  Synthetic DNA/RNA construct

<400>  167
ucucggcugg ugcccaagct t                                                21


<210>  168
<211>  21
<212>  DNA
<213>  Artificial Sequence

<220>
<223>  Synthetic DNA/RNA construct

<400>  168
cgugcgagca gccaaucaat t                                                21


<210>  169
<211>  21
<212>  DNA
<213>  Artificial Sequence

<220>
<223>  Synthetic DNA/RNA construct

<400>  169
ugugugucac caccccggct t                                                21


<210>  170
<211>  21
<212>  DNA
<213>  Artificial Sequence

<220>
<223>  Synthetic DNA/RNA construct

<400>  170
ucuucucagc caauggaggt t                                                21


<210>  171
<211>  21
<212>  DNA
<213>  Artificial Sequence

<220>
<223>  Synthetic DNA/RNA construct

<400>  171
ugcuucuccg guugcugaat t                                                21

<210>  172
<211>  21
<212>  DNA
<213>  Artificial Sequence

<220>
<223>  Synthetic DNA/RNA construct

<400>  172
cuucuccggu ugcugaaggt t                                                21

<210>  173
<211>  21
<212>  DNA
<213>  Artificial Sequence

<220>
<223>  Synthetic DNA/RNA construct

<400>  173
agaggacucc caagccugct t                                                21

<210>  174
<211>  21
<212>  DNA
<213>  Artificial Sequence

<220>
<223>  Synthetic DNA/RNA construct

<400>  174
gaggacuccc aagccugcut t                                                21

<210>  175
<211>  21
<212>  DNA
<213>  Artificial Sequence

<220>
<223>  Synthetic DNA/RNA construct

<400>  175
cucggcuggu gcccaagcut t                                                21

<210>  176
<211>  21
<212>  DNA
<213>  Artificial Sequence

<220>
<223>  Synthetic DNA/RNA construct

<400>  176
cagaggacuc ccaagccugt t                                                21

<210>  177
<211>  21
<212>  DNA
<213>  Artificial Sequence

<220>
<223>  Synthetic DNA/RNA construct

<400>  177
ugacucgggu cagaugugct t                                                21


<210>  178
<211>  21
<212>  DNA
<213>  Artificial Sequence

<220>
<223>  Synthetic DNA/RNA construct

<400>  178
cuccgguugc ugaaggcgct t                                                21


<210>  179
<211>  21
<212>  DNA
<213>  Artificial Sequence

<220>
<223>  Synthetic DNA/RNA construct

<400>  179
cggagaucac acaccaggut t                                                21


<210>  180
<211>  21
<212>  DNA
<213>  Artificial Sequence

<220>
<223>  Synthetic DNA/RNA construct

<400>  180
auugcagggc uguaauucgt t                                                21


<210>  181
<211>  21
<212>  DNA
<213>  Artificial Sequence

<220>
<223>  Synthetic DNA/RNA construct

<400>  181
uugcagggcu guaauucgat t                                                21


<210>  182

<211>  21
<212>  DNA
<213>  Artificial Sequence

<220>
<223>  Synthetic DNA/RNA construct

<400>  182
cgagaaaagu ucuugugugt t                                                21


<210>  183
<211>  21
<212>  DNA
<213>  Artificial Sequence

<220>
<223>  Synthetic DNA/RNA construct

<400>  183
ugugugcaga ggacucccat t                                                21


<210>  184
<211>  21
<212>  DNA
<213>  Artificial Sequence

<220>
<223>  Synthetic DNA/RNA construct

<400>  184
agcuaggauc acagcuccct t                                                21


<210>  185
<211>  21
<212>  DNA
<213>  Artificial Sequence

<220>
<223>  Synthetic DNA/RNA construct

<400>  185
ucuccgguug cugaaggcgt t                                                21


<210>  186
<211>  21
<212>  DNA
<213>  Artificial Sequence

<220>
<223>  Synthetic DNA/RNA construct

<400>  186
aagcuaggau cacagcucct t                                                21


<210>  187
<211>  21
<212>  DNA
<213>  Artificial Sequence

<220>
<223>  Synthetic DNA/RNA construct

<400>  187
cagggcugua auucgacuct t                                                21


<210>  188
<211>  21
<212>  DNA
<213>  Artificial Sequence

<220>
<223>  Synthetic DNA/RNA construct

<400>  188
ugaaggcgcu gcgccccuut t                                                21


<210>  189
<211>  21
<212>  DNA
<213>  Artificial Sequence

<220>
<223>  Synthetic DNA/RNA construct

<400>  189
cuguaauucg acuccggugt t                                                21


<210>  190
<211>  21
<212>  DNA
<213>  Artificial Sequence

<220>
<223>  Synthetic DNA/RNA construct

<400>  190
aguucuugug ugcagaggat t                                                21


<210>  191
<211>  21
<212>  DNA
<213>  Artificial Sequence

<220>
<223>  Synthetic DNA/RNA construct

<400>  191
cuggggugcg ggagcggugt t                                                21


<210>  192
<211>  21
<212>  DNA
<213>  Artificial Sequence

<220>
<223>  Synthetic DNA/RNA construct

<400>  192
guucuugugu gcagaggact t                                                21


<210>  193
<211>  21
<212>  DNA
<213>  Artificial Sequence

<220>
<223>  Synthetic DNA/RNA construct

<400>  193
gcugacucgg gucagaugut t                                                21


<210>  194
<211>  21
<212>  DNA
<213>  Artificial Sequence

<220>
<223>  Synthetic DNA/RNA construct

<400>  194
caaagcagag agaaggccgt t                                                21


<210>  195
<211>  21
<212>  DNA
<213>  Artificial Sequence

<220>
<223>  Synthetic DNA/RNA construct

<400>  195
cacgcgccac ucagucucct t                                                21


<210>  196
<211>  21
<212>  DNA
<213>  Artificial Sequence

<220>
<223>  Synthetic DNA/RNA construct

<400>  196
gcuucuccgg uugcugaagt t                                                21


<210>  197
<211>  21
<212>  DNA
<213>  Artificial Sequence

<220>
<223>  Synthetic DNA/RNA construct

<400>  197
gcgggcggcu cugggcgaut t                                                21

<210>  198
<211>  21
<212>  DNA
<213>  Artificial Sequence

<220>
<223>  Synthetic DNA/RNA construct

<400>  198
acccgccagg cgcccagaat t                                                21


<210>  199
<211>  21
<212>  DNA
<213>  Synthetic DNA/RNA construct

<400>  199
ggcgcgugcg agcagccaat t                                                21


<210>  200
<211>  21
<212>  DNA
<213>  Artificial Sequence

<220>
<223>  Synthetic DNA/RNA construct

<400>  200
cucucggcug gugcccaagt t                                                21


<210>  201
<211>  21
<212>  DNA
<213>  Artificial Sequence

<220>
<223>  Synthetic DNA/RNA construct

<400>  201
agcucccacu ucugaugcct t                                                21


<210>  202
<211>  21
<212>  DNA
<213>  Artificial Sequence

<220>
<223>  Synthetic DNA/RNA construct

<400>  202
gggcgauucu ggacccgcct t                                                21


<210>  203
<211>  21
<212>  DNA
<213>  Artificial Sequence

<220>
<223>  Synthetic DNA/RNA construct

<400>  203
acagcuccca cuucugaugt t                                                21


<210>  204
<211>  21
<212>  DNA
<213>  Artificial Sequence

<220>
<223>  Synthetic DNA/RNA construct

<400>  204
gaugugcacg accgcgggct t                                                21


<210>  205
<211>  21
<212>  DNA
<213>  Artificial Sequence

<220>
<223>  Synthetic DNA/RNA construct

<400>  205
guuaauugca gggcuguaat t                                                21


<210>  206
<211>  21
<212>  DNA
<213>  Artificial Sequence

<220>
<223>  Synthetic DNA/RNA construct

<400>  206
uuccgggucu cuucugcaat t                                                21


<210>  207
<211>  21
<212>  DNA
<213>  Artificial Sequence

<220>
<223>  Synthetic DNA/RNA construct

<400>  207
cugaaggcgc ugcgccccut t                                                21


<210>  208
<211>  21
<212>  DNA
<213>  Artificial Sequence

<220>
<223>  Synthetic DNA/RNA construct

<400>  208
aaaguucuug ugugcagagt t                                                 21


<210>  209
<211>  21
<212>  DNA
<213>  Artificial Sequence

<220>
<223>  Synthetic DNA/RNA construct

<400>  209
cccacccacu cggagaucat t                                                 21


<210>  210
<211>  21
<212>  DNA
<213>  Artificial Sequence

<220>
<223>  Synthetic DNA/RNA construct

<400>  210
uccgggucuc uucugcaact t                                                 21


<210>  211
<211>  21
<212>  DNA
<213>  Artificial Sequence

<220>
<223>  Synthetic DNA/RNA construct

<400>  211
gaaaaguucu ugugugcagt t                                                 21


<210>  212
<211>  21
<212>  DNA
<213>  Artificial Sequence

<220>
<223>  Synthetic DNA/RNA construct

<400>  212
uggugcccaa gcugacucgt t                                                 21


<210>  213
<211>  21
<212>  DNA
<213>  Artificial Sequence

<220>
<223>  Synthetic DNA/RNA construct

<400>  213
ggcuggugcc caagcugact t                                                 21

<210>  214
<211>  21
<212>  DNA
<213>  Artificial Sequence

<220>
<223>  Synthetic DNA/RNA construct

<400>  214
agggcuguaa uucgacucct t                                                21


<210>  215
<211>  21
<212>  DNA
<213>  Artificial Sequence

<220>
<223>  Synthetic DNA/RNA construct

<400>  215
ucagaugugc acgaccgcgt t                                                21


<210>  216
<211>  21
<212>  DNA
<213>  Artificial Sequence

<220>
<223>  Synthetic DNA/RNA construct

<400>  216
acucggagau cacacaccat t                                                21


<210>  217
<211>  21
<212>  DNA
<213>  Artificial Sequence

<220>
<223>  Synthetic DNA/RNA construct

<400>  217
gggcaggcgc gugcgagcat t                                                21


<210>  218
<211>  21
<212>  DNA
<213>  Artificial Sequence

<220>
<223>  Synthetic DNA/RNA construct

<400>  218
cacuucugau gcccaagcat t                                                21


<210>  219

<211>  21
<212>  DNA
<213>  Artificial Sequence

<220>
<223>  Synthetic DNA/RNA construct

<400>  219
caggcgccca gaagccacut t                                                21


<210>  220
<211>  21
<212>  DNA
<213>  Artificial Sequence

<220>
<223>  Synthetic DNA/RNA construct

<400>  220
uucuugugug cagaggacut t                                                21


<210>  221
<211>  21
<212>  DNA
<213>  Artificial Sequence

<220>
<223>  Synthetic DNA/RNA construct

<400>  221
cccacucgga gaucacacat t                                                21


<210>  222
<211>  21
<212>  DNA
<213>  Artificial Sequence

<220>
<223>  Synthetic DNA/RNA construct

<400>  222
ggacucccaa gccugcucat t                                                21


<210>  223
<211>  21
<212>  DNA
<213>  Artificial Sequence

<220>
<223>  Synthetic DNA/RNA construct

<400>  223
cucgggucag augugcacgt t                                                21


<210>  224
<211>  21
<212>  DNA
<213>  Artificial Sequence

<220>
<223>  Synthetic DNA/RNA construct

<400>  224
ucgggucaga ugugcacgat t                                                21


<210>  225
<211>  21
<212>  DNA
<213>  Artificial Sequence

<220>
<223>  Synthetic DNA/RNA construct

<400>  225
aaagagccaa ucagaagcut t                                                21


<210>  226
<211>  21
<212>  DNA
<213>  Artificial Sequence

<220>
<223>  Synthetic DNA/RNA construct

<400>  226
cgggucagau gugcacgact t                                                21


<210>  227
<211>  21
<212>  DNA
<213>  Artificial Sequence

<220>
<223>  Synthetic DNA/RNA construct

<400>  227
gaaggccgcc uacccugugt t                                                21


<210>  228
<211>  21
<212>  DNA
<213>  Artificial Sequence

<220>
<223>  Synthetic DNA/RNA construct

<400>  228
cucggagauc acacaccagt t                                                21


<210>  229
<211>  21
<212>  DNA
<213>  Artificial Sequence

<220>
<223>  Synthetic DNA/RNA construct

<400>  229
uggggugcgg gagcggugct t                                                21


<210>  230
<211>  21
<212>  DNA
<213>  Artificial Sequence

<220>
<223>  Synthetic DNA/RNA construct

<400>  230
caccccgaga aaaguucuut t                                                21


<210>  231
<211>  21
<212>  DNA
<213>  Artificial Sequence

<220>
<223>  Synthetic DNA/RNA construct

<400>  231
cccgagaaaa guucuugugt t                                                21


<210>  232
<211>  21
<212>  DNA
<213>  Artificial Sequence

<220>
<223>  Synthetic DNA/RNA construct

<400>  232
cucaaagcag agagaaggct t                                                21


<210>  233
<211>  21
<212>  DNA
<213>  Artificial Sequence

<220>
<223>  Synthetic DNA/RNA construct

<400>  233
agcggugcgg agccggcuct t                                                21


<210>  234
<211>  21
<212>  DNA
<213>  Artificial Sequence

<220>
<223>  Synthetic DNA/RNA construct

<400>  234
cuccucgcgc cggucgcgut t                                                21

<210>  235
<211>  21
<212>  DNA
<213>  Artificial Sequence

<220>
<223>  Synthetic DNA/RNA construct

<400>  235
uucugaugcc caagcauuut t                                                21


<210>  236
<211>  21
<212>  DNA
<213>  Artificial Sequence

<220>
<223>  Synthetic DNA/RNA construct

<400>  236
ccacuucuga ugcccaagct t                                                21


<210>  237
<211>  21
<212>  DNA
<213>  Artificial Sequence

<220>
<223>  Synthetic DNA/RNA construct

<400>  237
ugcacgaccg cgggcggcut t                                                21


<210>  238
<211>  21
<212>  DNA
<213>  Artificial Sequence

<220>
<223>  Synthetic DNA/RNA construct

<400>  238
gggucagaug ugcacgacct t                                                21


<210>  239
<211>  21
<212>  DNA
<213>  Artificial Sequence

<220>
<223>  Synthetic DNA/RNA construct

<400>  239
augcuucucc gguugcugat t                                                21


<210>  240

<211>  21
<212>  DNA
<213>  Artificial Sequence

<220>
<223>  Synthetic DNA/RNA construct

<400>  240
ggcggcucug ggcgauucut t                                                21


<210>  241
<211>  21
<212>  DNA
<213>  Artificial Sequence

<220>
<223>  Synthetic DNA/RNA construct

<400>  241
caagcuagga ucacagcuct t                                                21


<210>  242
<211>  21
<212>  DNA
<213>  Artificial Sequence

<220>
<223>  Synthetic DNA/RNA construct

<400>  242
gugcaacuau gcuucuccgt t                                                21


<210>  243
<211>  21
<212>  DNA
<213>  Artificial Sequence

<220>
<223>  Synthetic DNA/RNA construct

<400>  243
ccaggucgcg ugcaacuaut t                                                21


<210>  244
<211>  21
<212>  DNA
<213>  Artificial Sequence

<220>
<223>  Synthetic DNA/RNA construct

<400>  244
uuuacacccc gagaaaagut t                                                21


<210>  245
<211>  21
<212>  DNA
<213>  Artificial Sequence

<220>
<223>  Synthetic DNA/RNA construct

<400>  245
cccgccaggc gcccagaagt t                                                21


<210>  246
<211>  21
<212>  DNA
<213>  Artificial Sequence

<220>
<223>  Synthetic DNA/RNA construct

<400>  246
gucaccaccc cggcggcugt t                                                21


<210>  247
<211>  21
<212>  DNA
<213>  Artificial Sequence

<220>
<223>  Synthetic DNA/RNA construct

<400>  247
ccaagcuagg aucacagcut t                                                21


<210>  248
<211>  21
<212>  DNA
<213>  Artificial Sequence

<220>
<223>  Synthetic DNA/RNA construct

<400>  248
agaucacaca ccaggucgct t                                                21


<210>  249
<211>  21
<212>  DNA
<213>  Artificial Sequence

<220>
<223>  Synthetic DNA/RNA construct

<400>  249
aggccgccua cccugugcut t                                                21


<210>  250
<211>  21
<212>  DNA
<213>  Artificial Sequence

<220>
<223>  Synthetic DNA/RNA construct

<400>  250
uucuggaccc gccaggcgct t                                                21


<210>  251
<211>  21
<212>  DNA
<213>  Artificial Sequence

<220>
<223>  Synthetic DNA/RNA construct

<400>  251
cagcucccac uucugaugct t                                                21


<210>  252
<211>  21
<212>  DNA
<213>  Artificial Sequence

<220>
<223>  Synthetic DNA/RNA construct

<400>  252
gggcggcucu gggcgauuct t                                                21


<210>  253
<211>  21
<212>  DNA
<213>  Artificial Sequence

<220>
<223>  Synthetic DNA/RNA construct

<400>  253
ugcugaaggc gcugcgccct t                                                21


<210>  254
<211>  21
<212>  DNA
<213>  Artificial Sequence

<220>
<223>  Synthetic DNA/RNA construct

<400>  254
uguuaauugc agggcuguat t                                                21


<210>  255
<211>  21
<212>  DNA
<213>  Artificial Sequence

<220>
<223>  Synthetic DNA/RNA construct

<400>  255
uucgucagau ucauauguut t                                                21

<210>  256
<211>  21
<212>  DNA
<213>  Artificial Sequence

<220>
<223>  Synthetic DNA/RNA construct

<400>  256
gcugggugc gggagcggut t                                                21


<210>  257
<211>  21
<212>  DNA
<213>  Artificial Sequence

<220>
<223>  Synthetic DNA/RNA construct

<400>  257
ggcacgcgcc acucagucut t                                               21


<210>  258
<211>  21
<212>  DNA
<213>  Artificial Sequence

<220>
<223>  Synthetic DNA/RNA construct

<400>  258
aggucgcgug caacuaugct t                                               21


<210>  259
<211>  21
<212>  DNA
<213>  Artificial Sequence

<220>
<223>  Synthetic DNA/RNA construct

<400>  259
gacucggguc agaugugcat t                                               21


<210>  260
<211>  21
<212>  DNA
<213>  Artificial Sequence

<220>
<223>  Synthetic DNA/RNA construct

<400>  260
cgggcggcuc ugggcgauut t                                               21


<210>  261

<211>  21
<212>  DNA
<213>  Artificial Sequence

<220>
<223>  Synthetic DNA/RNA construct

<400>  261
gugcccaagc ugacucgggt t                                                 21


<210>  262
<211>  21
<212>  DNA
<213>  Artificial Sequence

<220>
<223>  Synthetic DNA/RNA construct

<400>  262
ggcucucggc uggugcccat t                                                 21


<210>  263
<211>  21
<212>  DNA
<213>  Artificial Sequence

<220>
<223>  Synthetic DNA/RNA construct

<400>  263
uuuuacaccc cgagaaaagt t                                                 21


<210>  264
<211>  21
<212>  DNA
<213>  Artificial Sequence

<220>
<223>  Synthetic DNA/RNA construct

<400>  264
cccagaagcc acucaaagct t                                                 21


<210>  265
<211>  21
<212>  DNA
<213>  Artificial Sequence

<220>
<223>  Synthetic DNA/RNA construct

<400>  265
ugcagggcug uaauucgact t                                                 21


<210>  266
<211>  21
<212>  DNA
<213>  Artificial Sequence

<220>
<223>  Synthetic DNA/RNA construct

<400>  266
gucagaugug cacgaccgct t                                                21


<210>  267
<211>  21
<212>  DNA
<213>  Artificial Sequence

<220>
<223>  Synthetic DNA/RNA construct

<400>  267
uaauugcagg gcuguaauut t                                                21


<210>  268
<211>  21
<212>  DNA
<213>  Artificial Sequence

<220>
<223>  Synthetic DNA/RNA construct

<400>  268
cgggucucuu cugcaacgut t                                                21


<210>  269
<211>  21
<212>  DNA
<213>  Artificial Sequence

<220>
<223>  Synthetic DNA/RNA construct

<400>  269
ucuggacccg ccaggcgcct t                                                21


<210>  270
<211>  21
<212>  DNA
<213>  Artificial Sequence

<220>
<223>  Synthetic DNA/RNA construct

<400>  270
cacacaccag gucgcgugct t                                                21


<210>  271
<211>  21
<212>  DNA
<213>  Artificial Sequence

<220>
<223>  Synthetic DNA/RNA construct

<400>  271
acacaccagg ucgcgugcat t                                                21

<210>  272
<211>  21
<212>  DNA
<213>  Artificial Sequence

<220>
<223>  Synthetic DNA/RNA construct

<400>  272
ugugucacca ccccggcggt t                                                21

<210>  273
<211>  21
<212>  DNA
<213>  Artificial Sequence

<220>
<223>  Synthetic DNA/RNA construct

<400>  273
gcgagcagcc aaucaaagat t                                                21

<210>  274
<211>  21
<212>  DNA
<213>  Artificial Sequence

<220>
<223>  Synthetic DNA/RNA construct

<400>  274
uaauucgacu ccggugugut t                                                21

<210>  275
<211>  21
<212>  DNA
<213>  Artificial Sequence

<220>
<223>  Synthetic DNA/RNA construct

<400>  275
cuucugaugc ccaagcauut t                                                21

<210>  276
<211>  21
<212>  DNA
<213>  Artificial Sequence

<220>
<223>  Synthetic DNA/RNA construct

<400>  276
agcagccaau caaagaguut t                                                21

<210>  277
<211>  21
<212>  DNA
<213>  Artificial Sequence

<220>
<223>  Synthetic DNA/RNA construct

<400>  277
ucugggcgau ucuggaccct t                                                21


<210>  278
<211>  21
<212>  DNA
<213>  Artificial Sequence

<220>
<223>  Synthetic DNA/RNA construct

<400>  278
guaauucgac uccggugugt t                                                21


<210>  279
<211>  21
<212>  DNA
<213>  Artificial Sequence

<220>
<223>  Synthetic DNA/RNA construct

<400>  279
ucuugugugc agaggacuct t                                                21


<210>  280
<211>  19
<212>  DNA
<213>  Artificial Sequence

<220>
<223>  Active target sequence

<400>  280
gcggccttct ctctgcttt                                                   19


<210>  281
<211>  19
<212>  DNA
<213>  Artificial Sequence

<220>
<223>  Active target sequence

<400>  281
cagaagagac ccggaaagg                                                   19


<210>  282

<211>  19
<212>  DNA
<213>  Artificial Sequence

<220>
<223>  Active target sequence

<400>  282
ccggagaagc atagttgca                                                    19


<210>  283
<211>  19
<212>  DNA
<213>  Artificial Sequence

<220>
<223>  Active target sequence

<400>  283
cgcgacctgg tgtgtgatc                                                    19


<210>  284
<211>  19
<212>  DNA
<213>  Artificial Sequence

<220>
<223>  Active target sequence

<400>  284
gagcaggctt gggagtcct                                                    19


<210>  285
<211>  19
<212>  DNA
<213>  Artificial Sequence

<220>
<223>  Active target sequence

<400>  285
tagttgcacg cgacctggt                                                    19


<210>  286
<211>  19
<212>  DNA
<213>  Artificial Sequence

<220>
<223>  Active target sequence

<400>  286
ggtccagaat cgcccagag                                                    19


<210>  287
<211>  19
<212>  DNA
<213>  Artificial Sequence

<220>
<223>  Active target sequence

<400>  287
gcagaagaga cccggaaag                                                    19


<210>  288
<211>  19
<212>  DNA
<213>  Artificial Sequence

<220>
<223>  Active target sequence

<400>  288
ccgcggtcgt gcacatctg                                                    19


<210>  289
<211>  19
<212>  DNA
<213>  Artificial Sequence

<220>
<223>  Active target sequence

<400>  289
cgggtccaga atcgcccag                                                    19


<210>  290
<211>  19
<212>  DNA
<213>  Artificial Sequence

<220>
<223>  Active target sequence

<400>  290
gccttcagca accggagaa                                                    19


<210>  291
<211>  19
<212>  DNA
<213>  Artificial Sequence

<220>
<223>  Active target sequence

<400>  291
acaccggagt cgaattaca                                                    19


<210>  292
<211>  19
<212>  DNA
<213>  Artificial Sequence

<220>
<223>  Active target sequence

<400>  292
aactttctc ggggtgtaa                                                     19


<210>  293
<211>  19
<212>  DNA
<213>  Artificial Sequence

<220>
<223>  Active target sequence

<400>  293
ttgggagtcc tctgcacac                                                    19


<210>  294
<211>  19
<212>  DNA
<213>  Artificial Sequence

<220>
<223>  Active target sequence

<400>  294
ttgggcacca gccgagagc                                                    19


<210>  295
<211>  19
<212>  DNA
<213>  Artificial Sequence

<220>
<223>  Active target sequence

<400>  295
tgcagaagag acccggaaa                                                    19


<210>  296
<211>  19
<212>  DNA
<213>  Artificial Sequence

<220>
<223>  Active target sequence

<400>  296
ccagaatcgc ccagagccg                                                    19


<210>  297
<211>  19
<212>  DNA
<213>  Artificial Sequence

<220>
<223>  Active target sequence

<400>  297
ggcgcagcgc cttcagcaa                                                    19

<210>  298
<211>  19
<212>  DNA
<213>  Artificial Sequence

<220>
<223>  Active target sequence

<400>  298
cacgcgacct ggtgtgtga                                                    19


<210>  299
<211>  19
<212>  DNA
<213>  Artificial Sequence

<220>
<223>  Active target sequence

<400>  299
gcgggtccag aatcgccca                                                    19


<210>  300
<211>  19
<212>  DNA
<213>  Artificial Sequence

<220>
<223>  Active target sequence

<400>  300
ttctctctgc tttgagtgg                                                    19


<210>  301
<211>  19
<212>  DNA
<213>  Artificial Sequence

<220>
<223>  Active target sequence

<400>  301
agccgagagc cggctccgc                                                    19


<210>  302
<211>  19
<212>  DNA
<213>  Artificial Sequence

<220>
<223>  Active target sequence

<400>  302
cacatctgac ccgagtcag                                                    19


<210>  303

<211>  19
<212>  DNA
<213>  Artificial Sequence

<220>
<223>  Active target sequence

<400>  303
gccgagagcc ggctccgca                                                    19


<210>  304
<211>  19
<212>  DNA
<213>  Artificial Sequence

<220>
<223>  Active target sequence

<400>  304
gcttgggcac cagccgaga                                                    19


<210>  305
<211>  19
<212>  DNA
<213>  Artificial Sequence

<220>
<223>  Active target sequence

<400>  305
ttgattggct gctcgcacg                                                    19


<210>  306
<211>  19
<212>  DNA
<213>  Artificial Sequence

<220>
<223>  Active target sequence

<400>  306
gccggggtgg tgacacaca                                                    19


<210>  307
<211>  19
<212>  DNA
<213>  Artificial Sequence

<220>
<223>  Active target sequence

<400>  307
cctccattgg ctgagaaga                                                    19


<210>  308
<211>  19
<212>  DNA
<213>  Artificial Sequence

<220>
<223>  Active target sequence

<400>  308
ttcagcaacc ggagaagca                                                    19


<210>  309
<211>  19
<212>  DNA
<213>  Artificial Sequence

<220>
<223>  Active target sequence

<400>  309
ccttcagcaa ccggagaag                                                    19


<210>  310
<211>  19
<212>  DNA
<213>  Artificial Sequence

<220>
<223>  Active target sequence

<400>  310
gcaggcttgg gagtcctct                                                    19


<210>  311
<211>  19
<212>  DNA
<213>  Artificial Sequence

<220>
<223>  Active target sequence

<400>  311
agcaggcttg ggagtcctc                                                    19


<210>  312
<211>  19
<212>  DNA
<213>  Artificial Sequence

<220>
<223>  Active target sequence

<400>  312
agcttgggca ccagccgag                                                    19


<210>  313
<211>  19
<212>  DNA
<213>  Artificial Sequence

<220>
<223>  Active target sequence

<400>  313
caggcttggg agtcctctg                                                    19


<210>  314
<211>  19
<212>  DNA
<213>  Artificial Sequence

<220>
<223>  Active target sequence

<400>  314
gcacatctga cccgagtca                                                    19


<210>  315
<211>  19
<212>  DNA
<213>  Artificial Sequence

<220>
<223>  Active target sequence

<400>  315
gcgccttcag caaccggag                                                    19


<210>  316
<211>  19
<212>  DNA
<213>  Artificial Sequence

<220>
<223>  Active target sequence

<400>  316
acctggtgtg tgatctccg                                                    19


<210>  317
<211>  19
<212>  DNA
<213>  Artificial Sequence

<220>
<223>  Active target sequence

<400>  317
cgaattacag ccctgcaat                                                    19


<210>  318
<211>  19
<212>  DNA
<213>  Artificial Sequence

<220>
<223>  Active target sequence

<400>  318
tcgaattaca gccctgcaa                                                    19

<210>  319
<211>  19
<212>  DNA
<213>  Artificial Sequence

<220>
<223>  Active target sequence

<400>  319
cacacaagaa cttttctcg                                                    19


<210>  320
<211>  19
<212>  DNA
<213>  Artificial Sequence

<220>
<223>  Active target sequence

<400>  320
tgggagtcct ctgcacaca                                                    19


<210>  321
<211>  19
<212>  DNA
<213>  Artificial Sequence

<220>
<223>  Active target sequence

<400>  321
gggagctgtg atcctagct                                                    19


<210>  322
<211>  19
<212>  DNA
<213>  Artificial Sequence

<220>
<223>  Active target sequence

<400>  322
cgccttcagc aaccggaga                                                    19


<210>  323
<211>  19
<212>  DNA
<213>  Artificial Sequence

<220>
<223>  Active target sequence

<400>  323
ggagctgtga tcctagctt                                                    19


<210>  324

<211>  19
<212>  DNA
<213>  Artificial Sequence

<220>
<223>  Active target sequence

<400>  324
gagtcgaatt acagccctg                                                    19


<210>  325
<211>  19
<212>  DNA
<213>  Artificial Sequence

<220>
<223>  Active target sequence

<400>  325
aaggggcgca gcgccttca                                                    19


<210>  326
<211>  19
<212>  DNA
<213>  Artificial Sequence

<220>
<223>  Active target sequence

<400>  326
caccggagtc gaattacag                                                    19


<210>  327
<211>  19
<212>  DNA
<213>  Artificial Sequence

<220>
<223>  Active target sequence

<400>  327
tcctctgcac acaagaact                                                    19


<210>  328
<211>  19
<212>  DNA
<213>  Artificial Sequence

<220>
<223>  Active target sequence

<400>  328
caccgctccc gcaccccag                                                    19


<210>  329
<211>  19
<212>  DNA
<213>  Artificial Sequence

<220>
<223>  Active target sequence

<400>  329
gtcctctgca cacaagaac                                                    19


<210>  330
<211>  19
<212>  DNA
<213>  Artificial Sequence

<220>
<223>  Active target sequence

<400>  330
acatctgacc cgagtcagc                                                    19


<210>  331
<211>  19
<212>  DNA
<213>  Artificial Sequence

<220>
<223>  Active target sequence

<400>  331
cggccttctc tctgctttg                                                    19


<210>  332
<211>  19
<212>  DNA
<213>  Artificial Sequence

<220>
<223>  Active target sequence

<400>  332
ggagactgag tggcgcgtg                                                    19


<210>  333
<211>  19
<212>  DNA
<213>  Artificial Sequence

<220>
<223>  Active target sequence

<400>  333
cttcagcaac cggagaagc                                                    19


<210>  334
<211>  19
<212>  DNA
<213>  Artificial Sequence

<220>
<223>  Active target sequence

<400>  334
atcgcccaga gccgcccgc                                                    19

<210>  335
<211>  19
<212>  DNA
<213>  Artificial Sequence

<220>
<223>  Active target sequence

<400>  335
ttctgggcgc ctggcgggt                                                    19

<210>  336
<211>  19
<212>  DNA
<213>  Artificial Sequence

<220>
<223>  Active target sequence

<400>  336
ttggctgctc gcacgcgcc                                                    19

<210>  337
<211>  19
<212>  DNA
<213>  Artificial Sequence

<220>
<223>  Active target sequence

<400>  337
cttgggcacc agccgagag                                                    19

<210>  338
<211>  19
<212>  DNA
<213>  Artificial Sequence

<220>
<223>  Active target sequence

<400>  338
ggcatcagaa gtgggagct                                                    19

<210>  339
<211>  19
<212>  DNA
<213>  Artificial Sequence

<220>
<223>  Active target sequence

<400>  339
ggcgggtcca gaatcgccc                                                    19

<210>  340
<211>  19
<212>  DNA
<213>  Artificial Sequence

<220>
<223>  Active target sequence

<400>  340
catcagaagt gggagctgt                                                    19


<210>  341
<211>  19
<212>  DNA
<213>  Artificial Sequence

<220>
<223>  Active target sequence

<400>  341
gcccgcggtc gtgcacatc                                                    19


<210>  342
<211>  19
<212>  DNA
<213>  Artificial Sequence

<220>
<223>  Active target sequence

<400>  342
ttacagccct gcaattaac                                                    19


<210>  343
<211>  19
<212>  DNA
<213>  Artificial Sequence

<220>
<223>  Active target sequence

<400>  343
ttgcagaaga gacccggaa                                                    19


<210>  344
<211>  19
<212>  DNA
<213>  Artificial Sequence

<220>
<223>  Active target sequence

<400>  344
aggggcgcag cgccttcag                                                    19


<210>  345

<211>  19
<212>  DNA
<213>  Artificial Sequence

<220>
<223>  Active target sequence

<400>  345
ctctgcacac aagaacttt                                                    19


<210>  346
<211>  19
<212>  DNA
<213>  Artificial Sequence

<220>
<223>  Active target sequence

<400>  346
tgatctccga gtgggtggg                                                    19


<210>  347
<211>  19
<212>  DNA
<213>  Artificial Sequence

<220>
<223>  Active target sequence

<400>  347
gttgcagaag agacccgga                                                    19


<210>  348
<211>  19
<212>  DNA
<213>  Artificial Sequence

<220>
<223>  Active target sequence

<400>  348
ctgcacacaa gaacttttc                                                    19


<210>  349
<211>  19
<212>  DNA
<213>  Artificial Sequence

<220>
<223>  Active target sequence

<400>  349
cgagtcagct tgggcacca                                                    19


<210>  350
<211>  19
<212>  DNA
<213>  Artificial Sequence

<220>
<223>  Active target sequence

<400>  350
gtcagcttgg gcaccagcc                                                    19


<210>  351
<211>  19
<212>  DNA
<213>  Artificial Sequence

<220>
<223>  Active target sequence

<400>  351
ggagtcgaat tacagccct                                                    19


<210>  352
<211>  19
<212>  DNA
<213>  Artificial Sequence

<220>
<223>  Active target sequence

<400>  352
cgcggtcgtg cacatctga                                                    19


<210>  353
<211>  19
<212>  DNA
<213>  Artificial Sequence

<220>
<223>  Active target sequence

<400>  353
tggtgtgtga tctccgagt                                                    19


<210>  354
<211>  19
<212>  DNA
<213>  Artificial Sequence

<220>
<223>  Active target sequence

<400>  354
tgctcgcacg cgcctgccc                                                    19


<210>  355
<211>  19
<212>  DNA
<213>  Artificial Sequence

<220>
<223>  Active target sequence

<400>  355
tgcttgggca tcagaagtg                                                    19


<210>  356
<211>  19
<212>  DNA
<213>  Artificial Sequence

<220>
<223>  Active target sequence

<400>  356
agtggcttct gggcgcctg                                                    19


<210>  357
<211>  19
<212>  DNA
<213>  Artificial Sequence

<220>
<223>  Active target sequence

<400>  357
agtcctctgc acacaagaa                                                    19


<210>  358
<211>  19
<212>  DNA
<213>  Artificial Sequence

<220>
<223>  Active target sequence

<400>  358
tgtgtgatct ccgagtggg                                                    19


<210>  359
<211>  19
<212>  DNA
<213>  Artificial Sequence

<220>
<223>  Active target sequence

<400>  359
tgagcaggct tgggagtcc                                                    19


<210>  360
<211>  19
<212>  DNA
<213>  Artificial Sequence

<220>
<223>  Active target sequence

<400>  360
cgtgcacatc tgacccgag                                                    19

<210>  361
<211>  19
<212>  DNA
<213>  Artificial Sequence

<220>
<223>  Active target sequence

<400>  361
tcgtgcacat ctgacccga                                                    19


<210>  362
<211>  19
<212>  DNA
<213>  Artificial Sequence

<220>
<223>  Active target sequence

<400>  362
agcttctgat tggctcttt                                                    19


<210>  363
<211>  19
<212>  DNA
<213>  Artificial Sequence

<220>
<223>  Active target sequence

<400>  363
gtcgtgcaca tctgacccg                                                    19


<210>  364
<211>  19
<212>  DNA
<213>  Artificial Sequence

<220>
<223>  Active target sequence

<400>  364
cacagggtag gcggccttc                                                    19


<210>  365
<211>  19
<212>  DNA
<213>  Artificial Sequence

<220>
<223>  Active target sequence

<400>  365
ctggtgtgtg atctccgag                                                    19


<210>  366

<211>  19
<212>  DNA
<213>  Artificial Sequence

<220>
<223>  Active target sequence

<400>  366
gcaccgctcc cgcacccca                                                    19


<210>  367
<211>  19
<212>  DNA
<213>  Artificial Sequence

<220>
<223>  Active target sequence

<400>  367
aagaactttt ctcggggtg                                                    19


<210>  368
<211>  19
<212>  DNA
<213>  Artificial Sequence

<220>
<223>  Active target sequence

<400>  368
cacaagaact tttctcggg                                                    19


<210>  369
<211>  19
<212>  DNA
<213>  Artificial Sequence

<220>
<223>  Active target sequence

<400>  369
gccttctctc tgctttgag                                                    19


<210>  370
<211>  19
<212>  DNA
<213>  Artificial Sequence

<220>
<223>  Active target sequence

<400>  370
gagccggctc cgcaccgct                                                    19


<210>  371
<211>  19
<212>  DNA
<213>  Artificial Sequence

<220>
<223>  Active target sequence

<400>  371
acgcgaccgg cgcgaggag                                                    19


<210>  372
<211>  19
<212>  DNA
<213>  Artificial Sequence

<220>
<223>  Active target sequence

<400>  372
aaatgcttgg gcatcagaa                                                    19


<210>  373
<211>  19
<212>  DNA
<213>  Artificial Sequence

<220>
<223>  Active target sequence

<400>  373
gcttgggcat cagaagtgg                                                    19


<210>  374
<211>  19
<212>  DNA
<213>  Artificial Sequence

<220>
<223>  Active target sequence

<400>  374
agccgcccgc ggtcgtgca                                                    19


<210>  375
<211>  19
<212>  DNA
<213>  Artificial Sequence

<220>
<223>  Active target sequence

<400>  375
ggtcgtgcac atctgaccc                                                    19


<210>  376
<211>  19
<212>  DNA
<213>  Artificial Sequence

<220>
<223>  Active target sequence

<400>  376
tcagcaaccg gagaagcat                                                    19


<210>  377
<211>  19
<212>  DNA
<213>  Artificial Sequence

<220>
<223>  Active target sequence

<400>  377
agaatcgccc agagccgcc                                                    19


<210>  378
<211>  19
<212>  DNA
<213>  Artificial Sequence

<220>
<223>  Active target sequence

<400>  378
gagctgtgat cctagcttg                                                    19


<210>  379
<211>  19
<212>  DNA
<213>  Artificial Sequence

<220>
<223>  Active target sequence

<400>  379
cggagaagca tagttgcac                                                    19


<210>  380
<211>  19
<212>  DNA
<213>  Artificial Sequence

<220>
<223>  Active target sequence

<400>  380
atagttgcac gcgacctgg                                                    19


<210>  381
<211>  19
<212>  DNA
<213>  Artificial Sequence

<220>
<223>  Active target sequence

<400>  381
acttttctcg ggtgtaaa                                                     19

<210>  382
<211>  19
<212>  DNA
<213>  Artificial Sequence

<220>
<223>  Active target sequence

<400>  382
cttctgggcg cctggcggg                                                    19


<210>  383
<211>  19
<212>  DNA
<213>  Artificial Sequence

<220>
<223>  Active target sequence

<400>  383
cagccgccgg ggtggtgac                                                    19


<210>  384
<211>  19
<212>  DNA
<213>  Artificial Sequence

<220>
<223>  Active target sequence

<400>  384
agctgtgatc ctagcttgg                                                    19


<210>  385
<211>  19
<212>  DNA
<213>  Artificial Sequence

<220>
<223>  Active target sequence

<400>  385
gcgacctggt gtgtgatct                                                    19


<210>  386
<211>  19
<212>  DNA
<213>  Artificial Sequence

<220>
<223>  Active target sequence

<400>  386
agcacagggt aggcggcct                                                    19


<210>  387

<211>  19
<212>  DNA
<213>  Artificial Sequence

<220>
<223>  Active target sequence

<400>  387
gcgcctggcg ggtccagaa                                                    19


<210>  388
<211>  19
<212>  DNA
<213>  Artificial Sequence

<220>
<223>  Active target sequence

<400>  388
gcatcagaag tgggagctg                                                    19


<210>  389
<211>  19
<212>  DNA
<213>  Artificial Sequence

<220>
<223>  Active target sequence

<400>  389
gaatcgccca gagccgccc                                                    19


<210>  390
<211>  19
<212>  DNA
<213>  Artificial Sequence

<220>
<223>  Active target sequence

<400>  390
gggcgcagcg ccttcagca                                                    19


<210>  391
<211>  19
<212>  DNA
<213>  Artificial Sequence

<220>
<223>  Active target sequence

<400>  391
tacagccctg caattaaca                                                    19


<210>  392
<211>  19
<212>  DNA
<213>  Artificial Sequence

<220>
<223>  Active target sequence

<400>  392
aacatatgaa tctgacgaa                                                    19


<210>  393
<211>  19
<212>  DNA
<213>  Artificial Sequence

<220>
<223>  Active target sequence

<400>  393
accgctcccg caccccagc                                                    19


<210>  394
<211>  19
<212>  DNA
<213>  Artificial Sequence

<220>
<223>  Active target sequence

<400>  394
agactgagtg gcgcgtgcc                                                    19


<210>  395
<211>  19
<212>  DNA
<213>  Artificial Sequence

<220>
<223>  Active target sequence

<400>  395
gcatagttgc acgcgacct                                                    19


<210>  396
<211>  19
<212>  DNA
<213>  Artificial Sequence

<220>
<223>  Active target sequence

<400>  396
tgcacatctg acccgagtc                                                    19


<210>  397
<211>  19
<212>  DNA
<213>  Artificial Sequence

<220>
<223>  Active target sequence

<400>  397
aatcgcccag agccgcccg                                                    19


<210>  398
<211>  19
<212>  DNA
<213>  Artificial Sequence

<220>
<223>  Active target sequence

<400>  398
cccgagtcag cttgggcac                                                    19


<210>  399
<211>  19
<212>  DNA
<213>  Artificial Sequence

<220>
<223>  Active target sequence

<400>  399
tgggcaccag ccgagagcc                                                    19


<210>  400
<211>  19
<212>  DNA
<213>  Artificial Sequence

<220>
<223>  Active target sequence

<400>  400
cttttctcgg ggtgtaaaa                                                    19


<210>  401
<211>  19
<212>  DNA
<213>  Artificial Sequence

<220>
<223>  Active target sequence

<400>  401
gctttgagtg gcttctggg                                                    19


<210>  402
<211>  19
<212>  DNA
<213>  Artificial Sequence

<220>
<223>  Active target sequence

<400>  402
gtcgaattac agccctgca                                                    19

<210>  403
<211>  19
<212>  DNA
<213>  Artificial Sequence

<220>
<223>  Active target sequence

<400>  403
gcggtcgtgc acatctgac                                                    19


<210>  404
<211>  19
<212>  DNA
<213>  Artificial Sequence

<220>
<223>  Active target sequence

<400>  404
aattacagcc ctgcaatta                                                    19


<210>  405
<211>  19
<212>  DNA
<213>  Artificial Sequence

<220>
<223>  Active target sequence

<400>  405
acgttgcaga agagacccg                                                    19


<210>  406
<211>  19
<212>  DNA
<213>  Artificial Sequence

<220>
<223>  Active target sequence

<400>  406
ggcgcctggc gggtccaga                                                    19


<210>  407
<211>  19
<212>  DNA
<213>  Artificial Sequence

<220>
<223>  Active target sequence

<400>  407
gcacgcgacc tggtgtgtg                                                    19


<210>  408

<211>  19
<212>  DNA
<213>  Artificial Sequence

<220>
<223>  Active target sequence

<400>  408
tgcacgcgac ctggtgtgt                                                    19


<210>  409
<211>  19
<212>  DNA
<213>  Artificial Sequence

<220>
<223>  Active target sequence

<400>  409
ccgccggggt ggtgacaca                                                    19


<210>  410
<211>  19
<212>  DNA
<213>  Artificial Sequence

<220>
<223>  Active target sequence

<400>  410
tctttgattg gctgctcgc                                                    19


<210>  411
<211>  19
<212>  DNA
<213>  Artificial Sequence

<220>
<223>  Active target sequence

<400>  411
acacaccgga gtcgaatta                                                    19


<210>  412
<211>  19
<212>  DNA
<213>  Artificial Sequence

<220>
<223>  Active target sequence

<400>  412
aatgcttggg catcagaag                                                    19


<210>  413
<211>  19
<212>  DNA
<213>  Artificial Sequence

<220>
<223>  Active target sequence

<400>  413
aactctttga ttggctgct                                                    19


<210>  414
<211>  19
<212>  DNA
<213>  Artificial Sequence

<220>
<223>  Active target sequence

<400>  414
gggtccagaa tcgcccaga                                                    19


<210>  415
<211>  19
<212>  DNA
<213>  Artificial Sequence

<220>
<223>  Active target sequence

<400>  415
cacaccggag tcgaattac                                                    19


<210>  416
<211>  19
<212>  DNA
<213>  Artificial Sequence

<220>
<223>  Active target sequence

<400>  416
gagtcctctg cacacaaga                                                    19


<210>  417
<211>  20
<212>  DNA
<213>  Artificial Sequence

<220>
<223>  Primer sequence

<400>  417
cccttccagc aaacaggtga                                                   20


<210>  418
<211>  21
<212>  DNA
<213>  Artificial Sequence

<220>
<223>  Primer sequence

<400>  418
gaacgacggg ataacattgc g                                                21


<210>  419
<211>  21
<212>  DNA
<213>  Artificial Sequence

<220>
<223>  Primer sequence

<400>  419
atggacagga ctgaacgtct t                                                21


<210>  420
<211>  20
<212>  DNA
<213>  Artificial Sequence

<220>
<223>  Primer sequence

<400>  420
tccagcaggt cagcaaagaa                                                  20


<210>  421
<211>  21
<212>  DNA
<213>  Artificial Sequence

<220>
<223>  Synthetic DNA/RNA construct

<400>  421
acuacugagu gacaguagat t                                                21


<210>  422
<211>  21
<212>  DNA
<213>  Artificial Sequence

<220>
<223>  Synthetic DNA/RNA construct

<400>  422
ucuacuguca cucaguagut t                                                21


<210>  423
<211>  21
<212>  DNA
<213>  Artificial Sequence

<220>
<223>  Synthetic DNA/RNA construct

<400>  423
gcucugcacu ucucgacuut t                                                21

<210>  424
<211>  21
<212>  DNA
<213>  Artificial Sequence

<220>
<223>  Synthetic DNA/RNA construct

<400>  424
aagucgagaa gugcagagct t                                                21

## Description

### TECHNICAL FIELD

The present invention belongs to the technical field of nucleic acids, particularly as it relates to oligomeric nucleic acid molecules associated with gene activation, e.g., small activating nucleic acid molecules, a uses of small activating nucleic acid molecules in activating or upregulating the transcription of the *ATOH1* gene, and uses thereof for treating diseases or conditions related to ATOH1 protein deficiency or insufficiency, such as for uses in treating hearing loss.

### BACKGROUND

Hearing loss is a serious global disease affecting 360 million people worldwide. Hearing loss not only affects the quality of life of patients, but is also a heavy burden on the medical system. An effective treatment method for hearing loss is lacking and there is an urgent need to develop new methods of treatment (WHO, 2013).

Hearing loss is a manifestation of hearing dysfunction and is clinically classified into three types: conductive, sensorineural, and mixed hearing loss. In conductive hearing loss, lesions are found in outer or middle ear causing an obstruction to the transmission of sound waves to the inner ear. Sensorineural hearing loss is the most common type of hearing loss and caused by the loss of hair cells in the inner cochlea (Liberman 1990). Any factor that causes both conductive and sensorineural hearing loss is referred to as mixed hearing loss. The human inner ear contains about 75,000 sensory hair cells which detect sound and motion through the mechanically-sensitive stereocilia bundle which are mechanical sensors that convert motion signals into electrical signals. The sound transmission mechanism is the adaptive amplification of sound by the inner ear through the outer hair cells and synaptic chemical transmission of inner hair cells, converting mechanical vibration of sound into electrical impulses on auditory nerve fibers. The electrical impulses are further processed, encoded, and interpreted by auditory brainstem and auditory cortex, finally presenting auditory imagery to external acoustic vibrations at a cognitive level.

The factors influencing hearing loss are classified into congenital and acquired hearing losses. Congenital factors mainly refer to hearing loss of a newborn at or shortly after birth, which can be caused by infection or acquired disease of a pregnant woman during pregnancy, improper use of special drugs, the occurrence of complications during childbirth, and the like. Acquired factors may occur at any age, including infection, head and ear trauma, prolonged exposure to environments with excessive noise and ototoxic drugs, and aging which result in the deterioration of sensory hair cells of cochlea (Gurtler and Lalwani 2002). In normal populations, men have hearing loss beginning at about age 45, and women a few years later. Over 40% of the population over 50 years of age and 70% of the population over 70 years of age have clinically significant hearing loss. As a result of an aging of population, the incidence of deafness in the elderly has also increased. In addition, the loss of hair cells in the vestibular epithelial cells of the inner ear also leads to balance dysfunction, causing dizziness and vertigo.

The *ATOH1* genes, also known as *ATH1, HATH1, MATH-1* and b*HLHa14,* encode atonal bHLH transcription factor 1 proteins, and belong to members of the basic helix-loop-helix (bHLH) transcription factor family. ATOH1 has a positive regulation effect in regenerative processes of inner ear hair cell morphology and function of mammals (Zheng and Gao 2000). *ATOH1* was the first gene found to be expressed in the development of the inner ear (Bermingham et al. 1999), and it regulates the development of nerve cells, such as the spinal cord, inner ear hair cells, cerebellar neurons, proprioceptive system neurons, and non-nerve cells. Hair cell regeneration is important during the development of the inner ear and *ATOH1* promotes hair cell differentiation and regeneration.

Therefore, overexpressing *ATOH1* can provide a new therapy for hearing loss by promoting the regeneration of hair cells. In addition, *ATOH1* also functions as a tumor suppressor gene. *ATOH1* is a definite tumor suppressor gene in colorectal cancer, and overexpression of *ATOH1* can suppress tumor cell proliferation and growth of transplanted tumors (Kazanjian and Shroyer 2011).

Vector-mediated, e.g., viral and liposomal,) *ATOH1* gene therapy has been shown to promote hair cell regeneration and improve hearing in animal experiments (Richardson and Atkinson 2015). Some investigators have induced hair cells to differentiate and regenerate by mediating the overexpression of *ATOH1* prior to the collapse of the Organ of Corti (Kawamoto et al. 2003; Gubbels et al. 2008). The Raphael Group at The University of Michigan reported that aminoglycoside antibiotics caused the regeneration of hair cells with partial restoration of auditory function after overexpressing *ATOH1* in the inner ear of fully deaf domesticated guinea pigs (Izumikawa et al. 2005). In neonatal mice the overexpression of *ATOH1* can convert supporting cells into hair cells which can promote the entry of other cells, e.g., intestinal cells, into a differentiated state (Aragaki et al. 2008), and reduce the proliferation of colon cancer cells (Leow, Polakis, and Gao 2005).

This gene delivery method usually requires vectors, and the currently used gene transfection vectors are viruses and liposomes. The current gene therapy product, CGF166, a recombinant adenovirus-mediated ATOH1 gene therapy, is being tested in Phase I Clinical Trial to treat hearing loss (Novartis, ClinicalTrials.gov Identifier: NCT02132130). Although the expression levels of the viral-gene therapy system is high, problems with vector safety and immune response cannot be completely overcome and the applications of a viral-gene therapy system for use in a human presents certain challenges. Therefore, the development of new treatment method is of particular importance.

### SUMMARY

One objective of the present invention is to provide a small activating nucleic acid molecule based on RNA activation process, which can increase the expression of ATOH1 protein by activating or upregulating the transcription of *ATOH1* to promote the regeneration of inner ear hair cells, restore hearing, or treat a disease or condition related to ATOH1 protein deficiency or insufficiency, such as tumor formation.

Another objective of the present invention is to provide a composition or a formulation comprising a small activating nucleic acid molecule.

Another objective of the present invention is to provide a use of a small activating nucleic acid molecule or a composition or formulation comprising the same in preparing a drug for activating or upregulating the expression of *ATOH1* in a cell.

Yet another objective of the present invention is to provide a method for activating or upregulating the expression of *ATOH1* in a cell.

Yet another objective of the present invention is to provide a use of a small activating nucleic acid molecule or a composition or formulation thereof in the preparation of a drug for treating a disease or a condition related to *ATOH1* protein deficiency or insufficiency, or in a method of treating a disease or condition such as hearing loss related to ATOH1 protein deficiency or insufficiency.

Still yet nother objective of the present invention is to provide an isolated small activating nucleic acid molecule target site of the *ATOH1* gene, wherein the target site comprises or is selected from any continuous sequence of 16 to 35 nucleotides in length in any of sequences set forth in SEQ ID NO: 2 to SEQ ID NO: 5.

### Technical Solution

In one aspect of the present invention, a small activating nucleic acid molecule (e.g., a small activating RNA saRNA) activating or upregulating the expression of *ATOH1* in a cell is provided, wherein one strand of a small activating nucleic acid molecule has at least 75% homology or complementarity to any nucleic acid sequence of 16 to 35 nucleotides in length in the promoter region of *ATOH1,* thereby activating or upregulating the expression of the gene, wherein the promoter region comprises 637 nucleotides upstream of a transcription start site. Specifically, one strand of a small activating nucleic acid molecule comprises or is selected from a nucleic acid sequence having at least 75% (e.g., at least about 79%, about 80%, about 85%, about 90%, about 95%, about 99% or 100%) homology or complementarity to a continuous sequence of 16 to 35 nucleotides in length in positions
- 578 to -386 (hotspot 1, SEQ ID NO: 2), positions -339 to -291 (hotspot 2, SEQ ID NO: 3), positions
- 162 to -86 (hotspot 3, SEQ ID NO: 4) or positions -55 to -34 (hotspot 4, SEQ ID NO: 5) upstream of the transcription start site of the ATOH1 gene promoter. More specifically, one strand of a small
activating nucleic acid molecule of the present invention has at least 75% (e.g., at least about 79%, about 80%, about 85%, about 90%, about 95%, about 99% or about 100%) homology or complementarity to any nucleotide sequence selected from SEQ ID NOs: 280-416. In one specific embodiment, one strand of a small activating nucleic acid molecule of the present invention comprises a nucleic acid sequence having at least 75% (e.g., at least about 79%, about 80%, about 85%, about 90%, about 95%, about 99% or about 100%) homology or complementarity to any nucleotide sequence selected from SEQ ID NOs: 280-416. In another embodiment, one strand of a small activating nucleic acid molecule of the present invention consists of a nucleic acid sequence having at least 75% (e.g., at least about 79%, about 80%, about 85%, about 90%, about 95%, about 99% or about 100%) homology or complementarity to any nucleotide sequence selected from SEQ ID NOs: 280-416. In yet another embodiment, one strand of a small activating nucleic acid molecule of the present invention is a nucleic acid sequence having at least 75% (e.g., at least about 79%, about 80%, about 85%, about 90%, about 95%, about 99% or about 100%) homology or complementarity to any nucleotide sequence selected from SEQ ID NOs: 280-416.

A small activating nucleic acid molecule of the present invention comprises a double-stranded small activating nucleic acid molecule targeting the promoter region of the *ATOH1* gene comprising a first nucleic acid strand and a second nucleic acid strand, wherein the first nucleic acid strand has at least 75% (e.g., at least about 79%, about 80%, about 85%, about 90%, about 95%, about 99% or about 100%) homology or complementarity to any continuous sequence of 16 to 35 nucleotides in length in positions -578 to -386 (hotspot 1, SEQ ID NO: 2), positions -339 to -291 (hotspot 2, SEQ ID NO: 3), positions -162 to -86 (hotspot 3, SEQ ID NO: 4) or positions -55 to -34 (hotspot 4, SEQ ID NO: 5) away from the transcription start site of the *ATOH1* gene promoter, and the first nucleic acid strand and the second nucleic acid strand can complementarily form a double-stranded nucleic acid structure capable of activating the expression of *ATOH1* in a cell.

The first nucleic acid strand and the second nucleic acid strand of a small activating nucleic acid molecule of the present invention can be present on either two different nucleic acid strands or on the same nucleic acid strand. When the first nucleic acid strand and the second nucleic acid strand are located on two different strands, at least one strand of a small activating nucleic acid molecule can have overhangs at the 5' terminus and/or the 3' terminus, e.g. overhangs of 0 to 6 nucleotides in length at 3' terminus, such that the overhangs of 0, 1, 2, 3, 4, 5 or 6 nucleotides in length. Preferably, both strands of a small activating nucleic acid molecule of the present invention have overhangs; more preferably, the 3' terminus of both strands of a small activating nucleic acid molecule can have overhangs of 0 to 6 nucleotides in length, e.g., overhangs of 0, 1, 2, 3, 4, 5 or 6 nucleotides in length; most preferably overhangs of 2 or 3 nucleotides in length. Preferably, the nucleotide of the overhang is dT.

A small activating nucleic acid molecule of the present invention can also comprise a small activating nucleic acid molecule capable of forming a double-stranded region hairpin structure, e.g., a single-stranded small activating RNA molecule. In one embodiment, a small activating nucleic acid molecule of the present invention comprises a single-stranded small activating RNA molecule targeting the promoter region of the *ATOH1* gene, wherein the single-stranded small activating nucleic acid molecule can form a double-stranded region hairpin structure. Preferably,when the first nucleic acid strand and the second nucleic acid strand are present on the same nucleic acid strand, a small activating nucleic acid molecule of the present invention can be a hairpin single-stranded nucleic acid molecule, wherein the first nucleic acid strand and the second nucleic acid strand have complementary regions capable of forming a double-stranded nucleic acid structure, and the double-stranded nucleic acid structure can promote the expression of *ATOH1* in a cell with, for example, a RNA activation mechanism.

In the aforementioned small activating nucleic acid molecule, the first nucleic acid strand and the second nucleic acid strand can have 16 to 35 nucleotides (e.g., 16, 17, 18, 19, 20, 21, 22, 23, 24, 25, 26, 27, 28, 29, 30, 31, 32, 33, 34 or 35 nucleotides) in length.

In one embodiment, the first nucleic acid strand of a small activating nucleic acid molecule of the present invention has at least 75% (e.g., at least about 79%, about 80%, about 85%, about 90%, about 95%, about 99% or about 100%) identity or homology to any nucleotide sequence selected from SEQ ID NOs: 6-142, and the second nucleic acid strand of a small activating nucleic acid molecule has at least 75% (e.g., at least about 79%, about 80%, about 85%, about 90%, about 95%, about 99% or about 100%) identity or homology to any nucleotide sequence selected from SEQ ID NOs: 143-279. In one embodiment, the first nucleic acid strand of a small activating nucleic acid molecule of the present invention comprises a nucleic acid sequence having at least 75% (e.g., at least about 79%, about 80%, about 85%, about 90%, about 95%, about 99% or about 100%) identity or homology to any nucleotide sequence selected from SEQ ID NOs: 6-142, or consists of a nucleic acid sequence having at least 75% (e.g., at least about 79%, about 80%, about 85%, about 90%, about 95%, about 99% or about 100%) identity or homology to any nucleotide sequence selected from SEQ ID NOs: 6-142; the second nucleic acid strand of a small activating nucleic acid molecule of the present invention comprises a nucleic acid sequence having at least 75% (e.g., at least about 79%, about 80%, about 85%, about 90%, about 95%, about 99% or about 100%) identity or homology to any nucleotide sequence selected from SEQ ID NOs: 143-279, or consists of a nucleic acid sequence having at least 75% (e.g., at least about 79%, about 80%, about 85%, about 90%, about 95%, about 99% or about 100%) identity or homology to any nucleotide sequence selected from SEQ ID NOs: 143-279. In a specific embodiment, the first nucleic acid strand of a small activating nucleic acid molecule of the present invention can comprise or be selected from any nucleotide sequence set forth in SEQ ID NOs: 6-142, and the second strand can comprise or be selected from any nucleotide sequence set forth in SEQ ID NOs: 143-279. In one embodiment, a small activating nucleic acid molecule described herein can be synthesized, transcribed *in vitro* or expressed by a vector.

All the nucleotides in a small activating nucleic acid molecule described herein can be natural non-chemically modified nucleotides or can comprise at least one modification. In one embodiment, the modification in a small activating nucleic acid molecule described herein can comprise chemical modification, for example, at least one nucleotide can have chemical modification, and the chemical modification used in the present invention can comprise or be selected from one or more or any combination of the following modifications:
(1) modification of a phosphodiester bond of nucleotides in the nucleotide sequence of a small activating nucleic acid molecule;
(2) modification of 2'-OH of a ribose in the nucleotide sequence of a small activating nucleic acid molecule;
(3) modification of a base in the nucleotide sequence of a small activating nucleic acid molecule; and
(4) at least one nucleotide in the nucleotide sequence of a small activating nucleic acid molecule being a locked nucleic acid.

The chemical modifications described herein is well-known to those skilled in the art, and the modification of the phosphodiester bond refers to the modification of oxygen in the phosphodiester bond, including, but not limited to, phosphorothioate modification and boranophosphate modification. Both modifications can stabilize an saRNA structure and maintain high specificity and high affinity for base pairing.

The ribose modification refers to the modification of 2'-OH in pentose of a nucleotide, i.e., the introduction of some substituents into hydroxyl positions of the ribose, for example, including but not limited to 2'-fluoro modification, 2'-oxymethyl modification, 2'-oxyethylidene methoxy modification, 2,4'-dinitrophenol modification, locked nucleic acid (LNA), 2'-amino modification, 2'-deoxy modification, and the like.

The base modification refers to the modification of the base of a nucleotide, for example, including but not limited to 5'-bromouracil modification, 5'-iodouracil modification, N-methyluracil modification, 2,6-diaminopurine modification, and the like.

These modifications can increase the bioavailability of a small activating nucleic acid molecule, improve affinity to a target sequence and enhance resistance to nuclease hydrolysis in a cell.

In addition, in order to promote the access of a small activating nucleic acid molecule into a cell, on the basis of the aforementioned modifications, a lipophilic group, such as cholesterol, can be introduced on the terminus of the first nucleic acid strand and/or the second nucleic acid strand of a small activating nucleic acid molecule to facilitate the interaction with the promoter region of a gene in the cell nucleus, as the cell membrane and nuclear membrane are composed of lipid bilayers.

After contacting and entering a cell, a small activating nucleic acid molecule disclosed herein can effectively activate or up-regulate the expression of ATOH1 gene in a cell, preferably up-regulate the expression by at least 10%.

Another aspect of the present invention also relates to a nucleic acid encoding a small activating nucleic acid molecule of the invention. In one embodiment, the nucleic acid is a DNA molecule.

Another aspect of the present invention provides a cell comprising the aforementioned small activating nucleic acid molecule or the nucleic acid encoding a small activating nucleic acid molecule described herein. In one embodiment, a small activating nucleic acid molecule of the present invention can be a double-stranded small activating nucleic acid molecule targeting the promoter region of the *ATOH1* gene, which comprises a first nucleic acid strand and a second nucleic acid strand. In another embodiment, a small activating nucleic acid molecule of the present invention can be a single-stranded small activating nucleic acid molecule targeting the promoter region of *ATOH1* gene.

Another aspect of the present invention provides a composition (e.g., a pharmaceutical composition). The composition comprises a small activating nucleic acid molecule of the present invention or a nucleic acid encoding a small activating nucleic acid molecule described herein and, optionally, a pharmaceutically acceptable carrier. In one embodiment, the pharmaceutically acceptable carrier can comprise or be selected from a liposome, a high-molecular polymer, and a polypeptide.

In another aspect of the present invention, a formulation is provided which comprises a small activating nucleic acid molecule described in the present invention, a nucleic acid encoding a small activating nucleic acid molecule described in the present invention, or a cell comprising a small activating nucleic acid molecule of the present invention or a nucleic acid encoding a small activating nucleic acid molecule of the present invention, or a composition comprising a small activating nucleic acid molecule of the present invention.

In another aspect of the present invention, a kit is provided, which comprises a small activating nucleic acid molecule of the present invention, a nucleic acid encoding a small activating nucleic acid molecule described herein, or a cell comprising a small activating nucleic acid molecule of the present invention or a nucleic acid encoding a small activating nucleic acid molecule of the present invention, or a composition comprising a small activating nucleic acid molecule of the present invention.

Another aspect of the present invention relates to uses of a small activating nucleic acid molecule of the present invention, the nucleic acid encoding a small activating nucleic acid molecule of the present invention, a cell comprising a small activating nucleic acid molecule of the present invention, or the nucleic acid encoding a small activating nucleic acid molecule of the present invention, or a composition comprising a small activating nucleic acid molecule of the present invention in the preparation of a drug or formulation for activating or upregulating the expression of *ATOH1* in a cell.

Another aspect of the present invention also relates to a method for activating or upregulating the expression of *ATOH1* in a cell. The method comprises administering a small activating nucleic acid molecule of the present invention, the nucleic acid encoding a small activating nucleic acid molecule of the present invention, or the composition or formulation comprising a small activating nucleic acid molecule of the present invention to the cell. In one embodiment, the method for activating/up-regulating the expression of *ATOH1* gene in a cell comprises administering a small activating nucleic acid molecule described in the present invention, the nucleic acid encoding a small activating nucleic acid molecule of the present invention, or the composition or formulation comprising a small activating nucleic acid molecule of the present invention to the cell wherein the cell includes, but not limited to, a cancer cell, a neural cell, e.g., a spinal cord cell, an inner ear hair cell, an inner ear support cell, a cerebellar neuron, a proprioceptive system neuron, a non-neural cell, and the like. In one embodiment, the cancer cell can be a cell from a solid tumor, e.g., liver cancer, lung cancer, bladder cancer, prostate cancer, and glioma. The aforementioned cell can be *in vitro,* e.g., a cell line or a cell strain, or can be present in a mammalian body, e.g., a human body.

A small activating nucleic acid molecule of the present invention can be directly introduced into a cell, or can be produced in the cell after a nucleotide sequence encoding a small activating nucleic acid molecule of the present invention is introduced into the cell. The cell is preferably a mammalian cell, more preferably a human cell. The aforementioned cell can be *in vitro,* such as a cell line or a cell strain, or can be present in a mammalian body, e.g., a human body. The human body can be a patient suffering from a disease or condition related to insufficient or decreased expression of the ATOH1 protein. A small activating nucleic acid molecule of the present invention can be administered at a sufficient dose to treat the disease or condition related to a deficiency of the ATOH1 protein or insufficient or decreased expression of ATOH1 protein. Specifically, the disease or condition related to deficiency of ATOH1 protein amount or insufficient or decreased expression of ATOH1 protein can comprise, for example, tumors, e.g., solid tumors, and the solid tumors can comprise, for example, liver cancer, lung cancer, bladder cancer, prostatic cancer, glioma, and the like.

Another aspect of the present invention provides an isolated small activating nucleic acid molecule acting site of the *ATOH1* gene, which has any continuous sequence of 16 to 35 nucleotides in length in the promoter region of *ATOH1* gene, and preferably, the acting site comprises or is selected from any continuous sequence of 16 to 35 nucleotides in length in any of the sequences set forth in SEQ ID NOs: 2-5. Specifically, the acting site can comprise or be selected from any nucleotide sequence set forth in SEQ ID NOs: 280-416.

Another aspect of the present invention relates to a method for treating a disease or condition related to insufficient or decreased expression of the ATOH1 protein in a subject, which comprises administering a small activating nucleic acid molecule of the present invention, a nucleic acid encoding a small activating nucleic acid molecule of the present invention, a cell comprising a small activating nucleic acid molecule of the present invention or a nucleic acid encoding a small activating nucleic acid molecule of the present invention or the composition comprising a small activating nucleic acid molecule of the present invention at a curative dose to the subject. In one embodiment, a method for treating a disease or condition related to insufficient or decreased expression of the ATOH1 protein in a subject in the present invention comprises administering a small activating nucleic acid molecule of the present invention, the nucleic acid encoding a small activating nucleic acid molecule of the present invention, the cell comprising a small activating nucleic acid molecule of the present invention or a nucleic acid encoding a small activating nucleic acid molecule of the present invention or a composition comprising a small activating nucleic acid molecule of the present invention, and a low-molecular-weight compound, antibody, polypeptide, or protein at a curative dose to the subject. The subject can be a mammal, e.g., as a human. In one embodiment, a disease or condition related to insufficient or decreased expression of the ATOH1 protein can comprise, e.g., hearing loss and tumor diseases, e.g., colorectal cancer.

Another aspect of the present invention relates to a method for treating hearing loss in a subject, which comprises administering a small activating nucleic acid molecule of the present invention, a nucleic acid encoding a small activating nucleic acid molecule of the present invention, a cell comprising a small activating nucleic acid molecule of the present invention, or a nucleic acid encoding a small activating nucleic acid molecule of the present invention, or a composition or formulation comprising a small activating nucleic acid molecule of the present invention at a curative dose to the subject. In one embodiment, the method for treating hearing loss in a subject comprises administering a small activating nucleic acid molecule of the present invention, the nucleic acid encoding a small activating nucleic acid molecule of the present invention, the cell comprising a small activating nucleic acid molecule of the present invention, or a nucleic acid encoding a small activating nucleic acid molecule of the present invention, or the composition comprising a small activating nucleic acid molecule of the present invention, and a low-molecular-weight compound, antibody, polypeptide or protein at a curative dose to the subject. The subject can be a mammal, e.g., as a human. Hearing loss or hearing impairment refers to a partial or complete loss of hearing with less sensitivity to daily speech. In 1997, the World Health Organization (WHO) issued standards that divided the severity of hearing loss into 4 levels according to average hearing loss at four frequency points of 500 Hz, 1000 Hz, 2000 Hz and 4000 Hz: 26-40 dB is mild hearing loss, 41-60 dB is moderate hearing loss, 61-80 dB is severe hearing loss, and greater than 80 dB is extremely severe hearing loss.

Another aspect of the present invention relates to uses of a small activating nucleic acid molecule of the present invention, a nucleic acid encoding a small activating nucleic acid molecule of the present invention, a cell comprising a small activating nucleic acid molecule of the present invention or a nucleic acid encoding a small activating nucleic acid molecule of the present invention or a composition comprising a small activating nucleic acid molecule of the present invention in the preparation of a drug for treating a disease or condition related to insufficient or decreased expression of the ATOH1 protein. The subject can be a mammal, e.g., a human. In one embodiment, the disease related to insufficient or decreased expression of the ATOH1 protein can comprise, e.g.,, hearing loss and tumor diseases, e.g., colorectal cancer.

In one embodiment, uses of a small activating nucleic acid molecule described in the present invention, a nucleic acid encoding a small activating nucleic acid molecule of the present invention, a cell comprising a small activating nucleic acid molecule of the present invention or a nucleic acid encoding a small activating nucleic acid molecule of the present invention, or a composition comprising a small activating nucleic acid molecule of the present invention in the preparation of a drug for hearing loss.

Another aspect of the present invention relates to uses of a small activating nucleic acid molecule of the present invention, a nucleic acid encoding a small activating nucleic acid molecule of the present invention, a cell comprising a small activating nucleic acid molecule of the present invention or a nucleic acid encoding a small activating nucleic acid molecule of the present invention, or a composition or formulation comprising a small activating nucleic acid molecule of the present invention in the preparation of a drug or pharmaceutical composition for treating a disease or condition related to insufficient or decreased expression of ATOH1 protein.

In one embodiment, uses of a small activating nucleic acid molecule described in the present invention, a nucleic acid encoding a small activating nucleic acid molecule of the present invention, a cell comprising a small activating nucleic acid molecule of the present invention or a nucleic acid encoding a small activating nucleic acid molecule of the present invention, or a composition or formulation comprising a small activating nucleic acid molecule of the present invention in the preparation of a drug or pharmaceutical composition for treating hearing loss.

### Advantages of the Present Invention

A small activating nucleic acid molecule, such as a small activating RNA (saRNA) is capable of activating or upregulating the expression of the *ATOH1* gene provided by the present invention and can permanently activate the *ATOH1* gene, thereby efficiently and specifically upregulating or restoring the expression of the *ATOH1* gene and protein while featuring lower toxic and side effects, which can be used in treating a disease or symptom related to insufficient or decreased expression of the ATOH1 protein such as hearing loss, and in preparing a drug or formulation for treating the disease or symptom related to insufficient or decreased expression of the ATOH1 protein.

### BRIEF DESCRIPTION OF THE DRAWINGS

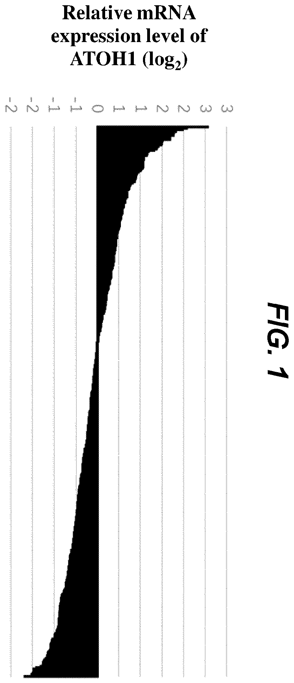

**FIG. 1** **shows changes in the expression of ATOH1 mRNA mediated by ATOH1 saRNA.** HEK293T cells were transfected by 403 saRNAs targeting the *ATOH1* promoter and mRNA expression of *ATOH1* was analyzed 72 h later using QuantiGene 2.0. Shown in the drawing are the changes in *ATOH1* mRNA expression relative to control treatment (mock) ranked from -637 to -19 according to the saRNA target positions in the ATOH1 promoter.

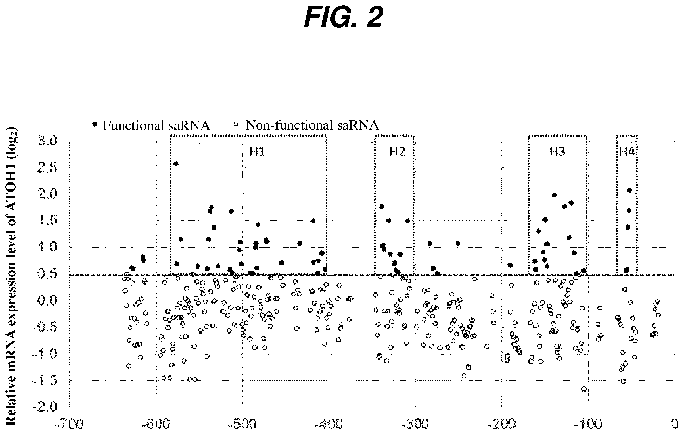

**FIG. 2** **shows the hotspot region of functional saRNA in the *ATOH1* promoter.** HEK293T cells were transfected by 403 saRNAs targeting ATOH1 promoter and mRNA expression of ATOH1 was analyzed 72 h later using QuantiGene 2.0. Shown in the drawing are changes in *ATOH1* mRNA expression relative to control treatment (mock) ranked from -637 to -19 according to the target positions of the saRNA in the *ATOH1* promoter. The dotted line in **FIG. 2** is the limit of 0.5 (log2) fold increase. The dotted frames show hotspot regions (H1-H4) for functional saRNAs .

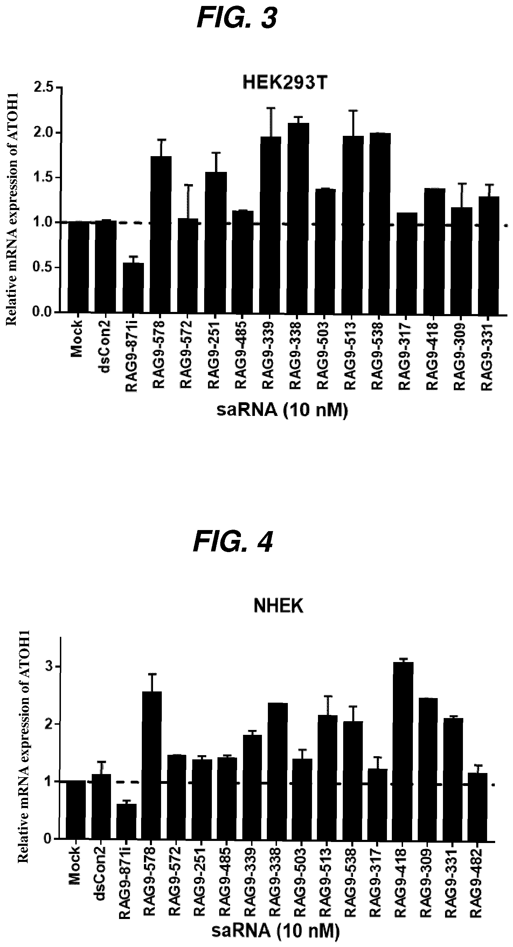

**FIG. 3** **shows the screening results for two-step RT-PCR validated by QuantiGene in HEK293T cells.** HEK293T cells were transfected with saRNA at a final concentration of 10 nM for 72 h. RNA was extracted using an Qiagen Rneasy kit and qPCR amplification was performed with a 7500FAST real-time PCR system after reverse transcription. An *HPRT1* gene was amplified as internal control. The relative mRNA expression values of *ATOH1* after treating cells with a single saRNA are shown. Mock, dsCon2, and RAG9-871i are represented as blank transfection, sequence-independent double-stranded RNA transfection, and small interference RNA control transfection, respectively.
**FIG. 4** **shows the screening results for two-step RT-PCR validated by Quantigene in NKEK cells.** Normal human epidermal keratinocytes (NHEK) cells were transfected with saRNA at a final concentration of 10 nM for 72 h. RNA was extracted using a Qiagen Rneasy kit and qPCR amplification was performed with a 7500FAST real-time PCR system after reverse transcription. An *HPRT1* gene was amplified as internal control. The relative mRNA expression values of *ATOH1* after treatment of cells with a single saRNA are shown. Mock, dsCon2, and RAG9-871i are represented as blank transfection, sequence-independent double-stranded RNA transfection, and small interference RNA control transfection, respectively.

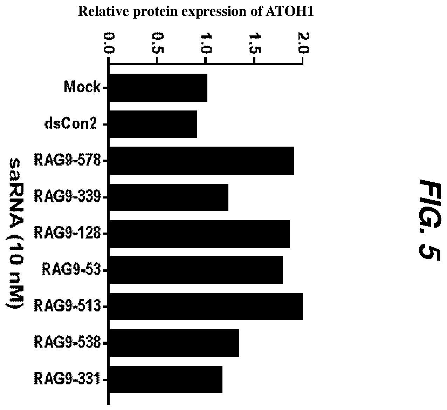

**FIG. 5** **shows *ATOH1* saRNA activating the expression of ATOH1 protein.** HEK293T cells were transfected with saRNA at a final concentration of 10 nM for 72 h. After collecting the cells and extracting total cell protein, ATOH1 protein expression was detected by Western Blot and α/β--tubulin was used as an internal control. The relative ATOH1 protein expression values after cell treatment with a single saRNA are shown. Mock, dsCon2, and RAG9-871i are represented as blank transfection, sequence-independent double-stranded RNA transfection, and small interference RNA control transfection, respectively.

### DETAILED DESCRIPTION

In the present invention, the related terms are defined as follows.

The term "complementarity" as used herein refers to the capability of forming base pairs between two oligonucleotide strands. The base pairs are generally formed through hydrogen bonds between nucleotides in the antiparallel oligonucleotide strands. The bases of the complementary oligonucleotide strands can be paired in the Watson-Crick manner (such as A to T, A to U, and C to G) or in any other manner allowing the formation of a duplex (such as Hoogsteen or reverse Hoogsteen base pairing).

Complementarity comprises complete complementarity and incomplete complementarity. "Complete complementarity" or "100% complementarity" means that each nucleotide from the first oligonucleotide strand can form a hydrogen bond with a nucleotide at a corresponding position in the second oligonucleotide strand in the double-stranded region of the double-stranded oligonucleotide molecule without "mispairing". "Incomplete complementarity" means that not all the nucleotide units of the two strands are bonded with each other by hydrogen bonds. For example, for two oligonucleotide strands each of 20 nucleotides in length in the double-stranded region, if only two base pairs in this double-stranded region can be formed through hydrogen bonds, the oligonucleotide strands have a complementarity of 10%. In the same example, if 18 base pairs in this double-stranded region can be formed through hydrogen bonds, the oligonucleotide strands have a complementarity of 90%. Substantial complementarity refers to at least about 75%, about 79%, about 80%, about 85%, about 90%, about 95%, about 99% or about 100% complementarity.

The term "oligonucleotide" as used herein refers to polymers of nucleotides, and includes, but is not limited to, single-stranded or double-stranded molecules of DNA, RNA, or DNA/RNA hybrid, oligonucleotide strands containing regularly and irregularly alternating deoxyribosyl portions and ribosyl portions, as well as modified and naturally or unnaturally existing frameworks for such oligonucleotides. The oligonucleotide for activating target gene transcription described herein is a small activating nucleic acid molecule.

The terms "oligonucleotide strand" and "oligonucleotide sequence" as used herein can be used interchangeably, referring to a generic term for short nucleotide sequences having less than 35 bases (including nucleotides in deoxyribonucleic acid (DNA) or ribonucleic acid (RNA)). In the present invention, an oligonucleotide strand can have any of 16 to 35 nucleotides in length.

As used herein, the term "first nucleic acid strand" can be a sense strand or an antisense strand. The sense strand of a small activating RNA refers to a nucleic acid strand contained in a small activating RNA duplex which has identity to the coding strand of the promoter DNA sequence of a target gene, and the antisense strand refers to a nucleic acid strand in a small activating RNA duplex which is complementary to the sense strand.

As used herein, the term "second nucleic acid strand" can also be a sense strand or an antisense strand. If the first oligonucleotide strand is a sense strand, the second oligonucleotide strand is an antisense strand; if the first oligonucleotide strand is an antisense strand, the second oligonucleotide strand is a sense strand.

The term "gene" as used herein refers to all nucleotide sequences required to encode a polypeptide chain or to transcribe a functional RNA. "Gene" can be an endogenous or fully or partially recombinant gene for a host cell (for example, because an exogenous oligonucleotide and a coding sequence for encoding a promoter are introduced into a host cell, or a heterogeneous promoter adjacent to an endogenous encoding sequence is introduced into a host cell). For example, the term "gene" comprises a nucleic acid sequence consisting of exons and introns. Protein-encoding sequences are, for example, sequences contained within exons in an open reading frame between an initiation codon and a termination codon, and as used herein, "gene" can comprise such as a gene regulatory sequence, such as a promoter, an enhancer, and all other sequences known in the art for controlling the transcription, expression or activity of another gene, no matter whether the gene comprises a coding sequence or a non-coding sequence. In one case, for example, "gene" can be used to describe a functional nucleic acid comprising a regulatory sequence such as a promoter or an enhancer. The expression of a recombinant gene can be controlled by one or more types of heterogeneous regulatory sequences.

The term "target gene" as used herein can refer to nucleic acid sequences naturally present in organisms, transgenes, viral or bacterial sequences, can be chromosomes or extrachromosomal genes, and/or can be transiently or stably transfected or incorporated into cells and/or chromatins thereof. The target gene can be a protein-encoding gene or a non-protein-encoding gene (such as a microRNA gene and a long non-coding RNA gene). The target gene generally contains a promoter sequence, and the positive regulation for the target gene can be achieved by designing a small activating nucleic acid molecule having sequence identity (also called homology) to the promoter sequence, characterized as the up-regulation of expression of the target gene. "Sequence of a target gene promoter" refers to a non-coding sequence of the target gene, and the reference of the sequence of a target gene promoter in the phrase "complementary to the sequence of a target gene promoter" of the present invention refers to a coding strand of the sequence, also known as a non-template strand, i.e., a nucleic acid sequence having the same sequence as the coding sequence of the gene. "Target" or "target sequence" refers to a sequence fragment in the sequence of a target gene promoter which is homologous or complementary with a sense oligonucleotide strand or an antisense oligonucleotide strand of a small activating nucleic acid molecule.

As used herein, the terms "sense strand" and "sense nucleic acid strand" can be used interchangeably, and the sense oligonucleotide strand of a small activating nucleic acid molecule refers to the first nucleic acid strand having identity to the coding strand of the sequence of a target gene promoter in a small activating nucleic acid molecule duplex.

As used herein, the terms "antisense strand" and "antisense nucleic acid strand" can be used interchangeably, and the antisense oligonucleotide strand of a small activating nucleic acid molecule refers to the second nucleic acid strand complementary with the sense oligonucleotide strand in a small activating nucleic acid molecule duplex.

The term "coding strand" as used herein refers to a DNA strand in the target gene which cannot be used for transcription, and the nucleotide sequence of this strand is the same as that of an RNA produced from transcription (in the RNA, T in DNA is replaced by U). The coding strand of the double-stranded DNA sequence of the target gene promoter described herein refers to a promoter sequence on the same DNA strand as the DNA coding strand of the target gene.

The term "template strand" as used herein refers to the other strand complementary with the coding strand in the double-stranded DNA of the target gene, i.e., the strand that, as a template, can be transcribed into RNA, and this strand is complementary with the transcribed RNA (A to U and G to C). In the process of transcription, RNA polymerase binds to the template strand, moves along the 3'→5' direction of the template strand, and catalyzes the synthesis of the RNA along the 5'→3' direction. The template strand of the double-stranded DNA sequence of the target gene promoter described herein refers to a promoter sequence on the same DNA strand as the DNA template strand of the target gene.

The term "promoter" as used herein refers to a sequence which plays a regulatory role for the transcription of a protein-coding or RNA-coding nucleic acid sequence by associating with them spatially. Generally, a eukaryotic gene promoter contains 100 to 5000 base pairs, although this length range is not intended to limit the term "promoter" as used herein. Although the promoter sequence is generally located at the 5' terminus of a protein-coding or RNA-coding sequence, the promoter sequence can also exist in exon and intron sequences.

The term "transcription start site" as used herein refers to a nucleotide marking the transcription start on the template strand of a gene. The transcription start site can appear on the template strand of the promoter region. A gene can have more than one transcription start site.

The term "identity" or "homology" as used herein means that one oligonucleotide strand (a sense or an antisense strand) of a small activating RNA has similarity to a coding strand or a template strand in a region of the promoter sequence of a target gene. As used herein, the "identity" or "homology" can be at least about 75%, about 79%, about 80%, about 85%, about 90%, about 95%, about 99% or about 100%.

The term "overhang" as used herein refers to non-base-paired nucleotides at the terminus (5' or 3') of an oligonucleotide strand, which is formed by one strand extending out of the other strand in a double-stranded oligonucleotide. A single-stranded region extending out of the 3' terminus and/or 5' terminus of a duplex is referred to as an overhang.

As used herein, the terms "gene activation", "activating gene expression", "gene up-regulation" and "up-regulating gene expression" can be used interchangeably, and mean an increase in transcription, translation, expression or activity of a certain nucleic acid as determined by measuring the transcriptional level, mRNA level, protein level, enzymatic activity, methylation state, chromatin state or configuration, translation level or the activity or state in a cell or biological system of a gene. These activities or states can be determined directly or indirectly. In addition, "gene activation", "activating gene expression", "gene up-regulation" or "up-regulating gene expression" refers to an increase in activity associated with a nucleic acid sequence, regardless of the mechanism of such activation. For example, the nucleic acid sequence plays a regulatory role as a regulatory sequence, the nucleic acid sequence is transcribed into RNA and the RNA is translated into a protein, thereby increasing the expression of the protein.

As used herein, the terms "small activating RNA", "saRNA", and "small activating nucleic acid molecule" can be used interchangeably, and refer to a nucleic acid molecule that can up-regulate target gene expression and can be composed of the first nucleic acid fragment (antisense nucleic acid strand, also referred to as antisense oligonucleotide strand) containing a nucleotide sequence having sequence identity or homology with the non-coding nucleic acid sequence (e.g., a promoter and an enhancer) of a target gene and the second nucleic acid fragment (sense nucleic acid strand, also referred to as sense oligonucleotide strand) containing a nucleotide sequence complementary with the first nucleic acid fragment, wherein the first nucleic acid fragment and the second nucleic acid fragment form a duplex. A small activating nucleic acid molecule can also be composed of a synthesized or vector-expressed single-stranded RNA molecule that can form a hairpin structure by two complementary regions within the molecule, wherein the first region comprises a nucleotide sequence having sequence identity to the target sequence of a promoter of a gene, and the second region comprises a nucleotide sequence which is complementary to the first region. The length of the duplex region of a small activating nucleic acid molecule is typically about 10 to about 50, about 12 to about 48, about 14 to about 46, about 16 to about 44, about 18 to about 42, about 20 to about 40, about 22 to about 38, about 24 to about 36, about 26 to about 34, and about 28 to about 32 base pairs, and typically about 10, about 15, about 20, about 25, about 30, about 35, about 40, about 45, or about 50 base pairs. In addition, the terms "saRNA", "small activating RNA", and "small activating nucleic acid molecule" also comprise nucleic acids other than the ribonucleotide, including, but not limited to, modified nucleotides or analogues.

As used herein, the term "hotspot" refers to a gene promoter region of at least 30 bp in length. The gathering of targets of functional small activating nucleic acid molecules appears in these hotspot regions, wherein at least 40% of a small activating nucleic acid molecules targeting these hotspot regions can induce a 1.1-fold or more change in the mRNA expression of a target gene.

As used herein, the term "synthesis" refers to a method for synthesis of an oligonucleotide, including any method allowing RNA synthesis, such as chemical synthesis, *in vitro* transcription, and/or vector-based expression.

According to the present invention, the expression of the *ATOH1* gene is up-regulated by RNA activation, and a related disease (particularly hearing loss) is treated by increasing the expression of the full-length ATOH1 protein. The *ATOH1* gene in the present invention is sometimes also called a target gene.

The method for preparing a small activating nucleic acid molecule provided by the present invention comprises sequence design and sequence synthesis.

The synthesis of the sequence of a small activating nucleic acid molecule of the present invention can be performed by adopting a chemical synthesis or can be entrusted to a biotechnology company specialized in nucleic acid synthesis.

Generally speaking, the chemical synthesis comprises the following four steps: (1) synthesis of oligomeric ribonucleotides, (2) deprotection, (3) purification and isolation; and (4) desalination and annealing.

For example, the specific steps for chemically synthesizing the saRNA described herein are as follows:

### (1) Synthesis of oligomeric ribonucleotides

Synthesis of 1 µM RNA was set in an automatic DNA/RNA synthesizer (e.g., Applied Biosystems EXPEDITE8909), and the coupling time of each cycle was also set as 10 to 15 min. With a solid phase-bonded 5'-O-p-dimethoxytriphenylmethyl-thymidine substrate as an initiator, one base was bonded to the solid phase substrate in the first cycle, and then, in the n^{th} (19 ≥ n ≥ 2) cycle, one base was bonded to the base bonded in the n-1^{th} cycle. This process was repeated until the synthesis of the whole nucleic acid sequence was completed.

### (2) Deprotection

The solid phase substrate bonded with the saRNA was put into a test tube, and 1 mL of a mixed solution of ethanol and ammonium hydroxide (volume ratio: 1:3) was added to the test tube. The test tube was then sealed and placed in an incubator, and the mixture was incubated at 25-70 °C for 2 to 30 h. The solution containing the solid phase substrate bonded with the saRNA was filtered, and the filtrate was collected. The solid phase substrate was rinsed with double distilled water twice (1 mL each time), and the filtrate was collected. The collected eluent was combined and dried under vacuum for 1 to 12 h. Then the solution was added with 1 mL of a solution of tetrabutylammonium fluoride in tetrahydrofuran (1 M), let stand at room temperature for 4 to 12 h, followed by addition of 2 mL of *n*-butanol. Precipitate was collected to give a single-stranded crude product of saRNA by high-speed centrifugation.

### (3) Purification and isolation

The resulting crude product of saRNA was dissolved in 2 mL of triethylamine acetate solution with a concentration of 1 mol/L, and the solution was separated by a reversed-phase C18 column of high pressure liquid chromatography to give a purified single-stranded product of saRNA.

### (4) Desalination and annealing

Salts were removed by gel filtration (size exclusion chromatography). A single sense oligomeric ribonucleic acid strand and a single antisense oligomeric ribonucleic acid strand were mixed in a 1 to 2 mL of buffer (10 mM Tris, pH = 7.5-8.0, 50 mM NaCl) at a molar ratio of 1:1. The solution was heated to 95 °C, and was then slowly cooled to room temperature to give a solution containing saRNA.

It was discovered in this study that after being introduced into a cell, the aforementioned saRNA could effectively increase the mRNA and protein expression of the full-length ATOH1.

The present invention will be further illustrated with reference to specific examples and drawings below. It should be understood that these examples are merely intended to illustrate the present invention rather than limit the scope of the present invention. In the following examples, study methods without specific conditions were generally in accordance with conventional conditions, such as conditions described in Sambrook, et al., Molecular Cloning: Laboratory Manual (New York: Cold Spring Harbor Laboratory Press, 1989), or conditions recommended by the manufacturer.

### EXAMPLES

### Example 1: Design and Synthesis of Small Activating Nucleic Acid Molecule Targeting ATOH1 Promoter

To screen functional saRNAs capable of activating *ATOH1* gene expression, a *ATOH1* promoter sequence with a length of 637 bps (-637 bp to -1 bp; SEQ ID NO: 1) was used as a template and an saRNA target with a lengths of 19 nucleotides were obtained by moving a base each time. Target sequences were filtered and the criteria for retaining target sequences were: (1) GC content between 30% and 80%, (2) with less than 5 continuous identical nucleotides, (3) with 3 or less dinucleotide repeat sequences; and (4) with 3 or less trinucleotide repeat sequences. There were 402 target sequences remaining after filtration and these entered the screening process as candidates. In addition, one target at the position -1407 bp of the *ATOH1* promoter was selected as a candidate target sequence, and the 403 target sequences were selected as candidates to enter screening process.

Each sense strand and each antisense strand in the double-stranded small activating RNAs used in the study were 21 nucleotides in length. The 19 nucleotides in the 5' region of the first nucleic acid strand, e.g., the sense strand of the double-stranded saRNA, had 100% identity to the target sequence of the promoter, and the 3' terminus of the first nucleic acid strand contained a TT sequence. The 19 nucleotides in the 5' region of the second nucleic acid strand were fully complementary with the first ribonucleic acid strand sequence, and the 3' terminus of the second nucleic acid strand contained a TT sequence. The aforementioned two strands of the double-stranded saRNA were mixed at a molar ratio of 1:1, and after annealing, and a double-stranded saRNA was formed. The sequence of the *ATOH1* promoter corresponds to position 1 to position 637 from 5' to 3' of SEQ ID No: 1 in the sequence listing:
- 637 aaatgcttgg gcatcagaag tgggagctgt gatcctagct tgggggcagc
- 587 acagggtagg cggccttctc tctgctttga gtggcttctg ggcgcctggc
- 537 gggtccagaa tcgcccagag ccgcccgcgg tcgtgcacat ctgacccgag
- 487 tcagcttggg caccagccga gagccggctc cgcaccgctc ccgcacccca
- 437 gccgccgggg tggtgacaca caccggagtc gaattacagc cctgcaatta
- 387 acatatgaat ctgacgaatt taaaagaagg aaaaaaaaaa aaaaacctga
- 337 gcaggcttgg gagtcctctg cacacaagaa cttttctcgg ggtgtaaaaa
- 287 ctctttgatt ggctgctcgc acgcgcctgc ccgcgccctc cattggctga
- 237 gaagacacgc gaccggcgcg aggagggggt tgggagagga gcggggggag
- 187 actgagtggc gcgtgccgct ttttaaaggg gcgcagcgcc ttcagcaacc
- 137 ggagaagcat agttgcacgc gacctggtgt gtgatctccg agtgggtggg
- 87 ggagggtcga ggagggaaaa aaaaataaga cgttgcagaa gagacccgga
- 37 aagggccttt tttttggttg agctggtgtc ccagtgc

### Example 2: High-throughput Screening of saRNAs Targeting ATOH1 Promoter

### (1) Cell culture and transfection

Normal Human Epidermal Keratinocytes (NHEK) and human renal embryonic cells (HEK293T) were cultured in DMEM media (Gibco). All the media contained 10% bovine calf serum (Sigma-Aldrich) and 1% penicillin/streptomycin (Gibco). The cells were cultured at 37 °C under 5% CO₂. According to the instructions provided by the manufacturer, RNAiMax (Invitrogen, Carlsbad, CA) was used to transfect small activating RNAs at a concentration of 10 nM (unless otherwise specified).

### (2) QuantiGene 2.0 assay

Cells were plated into a 96-well plate at 4000 cells/well and transfected with oligonucleotide duplexes, and 72 h after transfection, the mRNA levels of target genes were quantitatively determined with a QuantiGene 2.0 kit (AffyMetrix, product numbers were QGS-1000, ATOH1 Assay ID: SA-6000952, and HPRT Assay ID: SA-10030). QuantiGene 2.0 assay is based on hybridization technology, wherein mRNA levels were directly quantified with genespecific probes. The experimental procedure was briefly described as follows: a lysis solution was added to lyse the transfected cells, and the cell lysates were added into a capture well plate coated with probe for ATOH1 and HPRT1 (housekeeping gene) for hybridizing overnight at 55 °C. In order to enhance hybridization signal, hybridizations with 2.0 PreAMP Probe, 2.0 AMP Probe and 2.0 Label Probe (provided by Quantigene 2.0 kit) were conducted sequentially in 100 µL of a corresponding buffer solution (provided by Quantigene 2.0 kit). All the hybridizations were conducted with shaking at 50 to 55°C for 1 h. After the last wash step, 2.0 Substrate was added to the solution and incubated at room temperature for 5 min. Optical signals were detected with an Infinite 200 PRO plate reader (Tecan, Switzerland).

### (3) Screening of functional saRNAs

In order to obtain functional saRNAs capable of activating transcription of ATOH1, HEK293T cells were transfected with the above 403 saRNAs at a concentration of 10 nM, and light signals were analyzed 72 h later by the method described in QuantiGene. As shown in Table 1, 32 (7.9%) and 105 (26.1%) saRNAs showed high and mild activating activities, and 266 (66.0%) saRNAs had no influence on the expression of ATOH1. The maximum activation was 5.88 fold. These saRNAs with activating activity are referred to as functional saRNA.

**Table 1. High-throughput screening results of ATOH1**

| saRNA activity | log2 value (fold) of changes in ATOH1 | Number of saRNAs | Percentage |
|---|---|---|---|
| High activation | ≥ 1.00 (2.00) ~ ≤ 2.557 (5.883) | 32 | 7.9% |
| Mild activation | ≥ 0.14 (1.10) ~ < 1.00 (2.00) | 105 | 26.1% |
| No activating effect | < 0.14 (1.10) | 266 | 66.0% |
| Sum | | 403 | 100% |

**FIG. 1** further shows the activity distribution of the *ATOH1* saRNAs from high activation to high inhibition.

**Table 2. Active saRNA sequences, active target sequences thereof and resulting changes in mRNA expression of ATOH1**

| **saRNA** | **Active target sequence (5'-3')** | **Sense sequence (5'-3')** | **Antisense sequence (5'-3')** | **Fold of changes in relative mRNA expression of *ATOH1*** |
|---|---|---|---|---|
| RAG9-578 | GCGGCCTTCTCTCTGCTTT (SEQ ID NO: 280) | GCGGCCUUCUCUCUGCUUUTT (SEQ ID NO: 6) | AAAGCAGAGAGAAGGCCGCTT (SEQ ID NO: 143) | 5.88 |
| RAG9-52 | CAGAAGAGACCCGGAAAGG (SEQ ID NO: 281) | CAGAAGAGACCCGGAAAGGTT (SEQ ID NO: 7) | CCUUUCCGGGUCUCUUCUGTT (SEQ ID NO: 144) | 4.17 |
| RAG9-139 | CCGGAGAAGCATAGTTGCA (SEQ ID NO: 282) | CCGGAGAAGCAUAGUUGCATT (SEQ ID NO: 8) | UGCAACUAUGCUUCUCCGGTT (SEQ ID NO: 145) | 3.92 |
| RAG9-120 | CGCGACCTGGTGTGTGATC (SEQ ID NO: 283) | CGCGACCUGGUGUGUGAUCTT (SEQ ID NO: 9) | GAUCACACACCAGGUCGCGTT (SEQ ID NO: 146) | 3.53 |
| RAG9-339 | GAGCAGGCTTGGGAGTCCT (SEQ ID NO: 284) | GAGCAGGCUUGGGAGUCCUTT (SEQ ID NO: 10) | AGGACUCCCAAGCCUGCUCTT (SEQ ID NO: 147) | 3.41 |
| RAG9-128 | TAGTTGCACGCGACCTGGT (SEQ ID NO: 285) | UAGUUGCACGCGACCUGGUTT (SEQ ID NO: 11) | ACCAGGUCGCGUGCAACUATT (SEQ ID NO: 148) | 3.37 |
| RAG9-536 | GGTCCAGAATCGCCCAGAG (SEQ ID NO: 286) | GGUCCAGAAUCGCCCAGAGTT (SEQ ID NO: 12) | CUCUGGGCGAUUCUGGACCTT (SEQ ID NO: 149) | 3.37 |
| RAG9-53 | GCAGAAGAGACCCGGAAAG (SEQ ID NO: 287) | GCAGAAGAGACCCGGAAAGTT (SEQ ID NO: 13) | CUUUCCGGGUCUCUUCUGCTT (SEQ ID NO: 150) | 3.21 |
| RAG9-513 | CCGCGGTCGTGCACATCTG (SEQ ID NO: 288) | CCGCGGUCGUGCACAUCUGTT (SEQ ID NO: 14) | CAGAUGUGCACGACCGCGGTT (SEQ ID NO: 151) | 3.19 |
| RAG9-538 | CGGGTCCAGAATCGCCCAG (SEQ ID NO: 289) | CGGGUCCAGAAUCGCCCAGTT (SEQ ID NO: 15) | CUGGGCGAUUCUGGACCCGTT (SEQ ID NO: 152) | 3.18 |
| RAG9-150 | GCCTTCAGCAACCGGAGAA (SEQ ID NO: 290) | GCCUUCAGCAACCGGAGAATT (SEQ ID NO: 16) | UUCUCCGGUUGCUGAAGGCTT (SEQ ID NO: 153) | 2.83 |
| RAG9-418 | ACACCGGAGTCGAATTACA (SEQ ID NO: 291) | ACACCGGAGUCGAAUUACATT (SEQ ID NO: 17) | UGUAAUUCGACUCCGGUGUTT (SEQ ID NO: 154) | 2.81 |
| RAG9-309 | AACTTTTCTCGGGGTGTAA (SEQ ID NO: 292) | AACUUUUCUCGGGGUGUAATT (SEQ ID NO: 18) | UUACACCCCGAGAAAAGUUTT (SEQ ID NO: 155) | 2.79 |
| RAG9-331 | TTGGGAGTCCTCTGCACAC (SEQ ID NO: 293) | UUGGGAGUCCUCUGCACACTT (SEQ ID NO: 19) | GUGUGCAGAGGACUCCCAATT (SEQ ID NO: 156) | 2.79 |
| RAG9-482 | TTGGGCACCAGCCGAGAGC (SEQ ID NO: 294) | UUGGGCACCAGCCGAGAGCTT (SEQ ID NO: 20) | GCUCUCGGCUGGUGCCCAATT (SEQ ID NO: 157) | 2.66 |
| RAG9-54 | TGCAGAAGAGACCCGGAAA (SEQ ID NO: 295) | UGCAGAAGAGACCCGGAAATT (SEQ ID NO: 21) | UUUCCGGGUCUCUUCUGCATT (SEQ ID NO: 158) | 2.61 |
| RAG9-533 | CCAGAATCGCCCAGAGCCG (SEQ ID NO: 296) | CCAGAAUCGCCCAGAGCCGTT (SEQ ID NO: 22) | CGGCUCUGGGCGAUUCUGGTT (SEQ ID NO: 159) | 2.57 |
| RAG9-158 | GGCGCAGCGCCTTCAGCAA (SEQ ID NO: 297) | GGCGCAGCGCCUUCAGCAATT (SEQ ID NO: 23) | UUGCUGAAGGCGCUGCGCCTT (SEQ ID NO: 160) | 2.47 |
| RAG9-122 | CACGCGACCTGGTGTGTGA (SEQ ID NO: 298) | CACGCGACCUGGUGUGUGATT (SEQ ID NO: 24) | UCACACACCAGGUCGCGUGTT (SEQ ID NO: 161) | 2.26 |
| RAG9-539 | GCGGGTCCAGAATCGCCCA (SEQ ID NO: 299) | GCGGGUCCAGAAUCGCCCATT (SEQ ID NO: 25) | UGGGCGAUUCUGGACCCGCTT (SEQ ID NO: 162) | 2.21 |
| RAG9-572 | TTCTCTCTGCTTTGAGTGG (SEQ ID NO: 300) | UUCUCUCUGCUUUGAGUGGTT (SEQ ID NO: 26) | CCACUCAAAGCAGAGAGAATT (SEQ ID NO: 163) | 2.21 |
| RAG9-473 | AGCCGAGAGCCGGCTCCGC (SEQ ID NO: 301) | AGCCGAGAGCCGGCUCCGCTT (SEQ ID NO: 27) | GCGGAGCCGGCUCUCGGCUTT (SEQ ID NO: 164) | 2.19 |
| RAG9-502 | CACATCTGACCCGAGTCAG (SEQ ID NO: 302) | CACAUCUGACCCGAGUCAGTT (SEQ ID NO: 28) | CUGACUCGGGUCAGAUGUGTT (SEQ ID NO: 165) | 2.11 |
| RAG9-472 | GCCGAGAGCCGGCTCCGCA (SEQ ID NO: 303) | GCCGAGAGCCGGCUCCGCATT (SEQ ID NO: 29) | UGCGGAGCCGGCUCUCGGCTT (SEQ ID NO: 166) | 2.11 |
| RAG9-484 | GCTTGGGCACCAGCCGAGA (SEQ ID NO: 304) | GCUUGGGCACCAGCCGAGATT (SEQ ID NO: 30) | UCUCGGCUGGUGCCCAAGCTT (SEQ ID NO: 167) | 2.10 |
| RAG9-283 | TTGATTGGCTGCTCGCACG (SEQ ID NO: 305) | UUGAUUGGCUGCUCGCACGTT (SEQ ID NO: 31) | CGUGCGAGCAGCCAAUCAATT (SEQ ID NO: 168) | 2.09 |
| RAG9-434 | GCCGGGGTGGTGACACACA (SEQ ID NO: 306) | GCCGGGGUGGUGACACACATT (SEQ ID NO: 32) | UGUGUGUCACCACCCCGGCTT (SEQ ID NO: 169) | 2.09 |
| RAG9-251 | CCTCCATTGGCTGAGAAGA (SEQ ID NO: 307) | CCUCCAUUGGCUGAGAAGATT (SEQ ID NO: 33) | UCUUCUCAGCCAAUGGAGGTT (SEQ ID NO: 170) | 2.09 |
| RAG9-147 | TTCAGCAACCGGAGAAGCA (SEQ ID NO: 308) | UUCAGCAACCGGAGAAGCATT (SEQ ID NO: 34) | UGCUUCUCCGGUUGCUGAATT (SEQ ID NO: 171) | 2.07 |
| RAG9-149 | CCTTCAGCAACCGGAGAAG (SEQ ID NO: 309) | CCUUCAGCAACCGGAGAAGTT (SEQ ID NO: 35) | CUUCUCCGGUUGCUGAAGGTT (SEQ ID NO: 172) | 2.07 |
| RAG9-337 | GCAGGCTTGGGAGTCCTCT (SEQ ID NO: 310) | GCAGGCUUGGGAGUCCUCUTT (SEQ ID NO: 36) | AGAGGACUCCCAAGCCUGCTT (SEQ ID NO: 173) | 2.05 |
| RAG9-338 | AGCAGGCTTGGGAGTCCTC (SEQ ID NO: 311) | AGCAGGCUUGGGAGUCCUCTT (SEQ ID NO: 37) | GAGGACUCCCAAGCCUGCUTT (SEQ ID NO: 174) | 2.01 |
| RAG9-485 | AGCTTGGGCACCAGCCGAG (SEQ ID NO: 312) | AGCUUGGGCACCAGCCGAGTT (SEQ ID NO: 38) | CUCGGCUGGUGCCCAAGCUTT (SEQ ID NO: 175) | 1.98 |
| RAG9-336 | CAGGCTTGGGAGTCCTCTG (SEQ ID NO: 313) | CAGGCUUGGGAGUCCUCUGTT (SEQ ID NO: 39) | CAGAGGACUCCCAAGCCUGTT (SEQ ID NO: 176) | 1.92 |
| RAG9-503 | GCACATCTGACCCGAGTCA (SEQ ID NO: 314) | GCACAUCUGACCCGAGUCATT (SEQ ID NO: 40) | UGACUCGGGUCAGAUGUGCTT (SEQ ID NO: 177) | 1.90 |
| RAG9-152 | GCGCCTTCAGCAACCGGAG (SEQ ID NO: 315) | GCGCCUUCAGCAACCGGAGTT (SEQ ID NO: 41) | CUCCGGUUGCUGAAGGCGCTT (SEQ ID NO: 178) | 1.86 |
| RAG9-116 | ACCTGGTGTGTGATCTCCG (SEQ ID NO: 316) | ACCUGGUGUGUGAUCUCCGTT (SEQ ID NO: 42) | CGGAGAUCACACACCAGGUTT (SEQ ID NO: 179) | 1.85 |
| RAG9-408 | CGAATTACAGCCCTGCAAT (SEQ ID NO: 317) | CGAAUUACAGCCCUGCAAUTT (SEQ ID NO: 43) | AUUGCAGGGCUGUAAUUCGTT (SEQ ID NO: 180) | 1.84 |
| RAG9-409 | TCGAATTACAGCCCTGCAA (SEQ ID NO: 318) | UCGAAUUACAGCCCUGCAATT (SEQ ID NO: 44) | UUGCAGGGCUGUAAUUCGATT (SEQ ID NO: 181) | 1.83 |
| RAG9-317 | CACACAAGAACTTTTCTCG (SEQ ID NO: 319) | CACACAAGAACUUUUCUCGTT (SEQ ID NO: 45) | CGAGAAAAGUUCUUGUGUGTT (SEQ ID NO: 182) | 1.81 |
| RAG9-330 | TGGGAGTCCTCTGCACACA (SEQ ID NO: 320) | UGGGAGUCCUCUGCACACATT (SEQ ID NO: 46) | UGUGUGCAGAGGACUCCCATT (SEQ ID NO: 183) | 1.80 |
| RAG9-616 | GGGAGCTGTGATCCTAGCT (SEQ ID NO: 321) | GGGAGCUGUGAUCCUAGCUTT (SEQ ID NO: 47) | AGCUAGGAUCACAGCUCCCTT (SEQ ID NO: 184) | 1.75 |
| RAG9-151 | CGCCTTCAGCAACCGGAGA (SEQ ID NO: 322) | CGCCUUCAGCAACCGGAGATT (SEQ ID NO: 48) | UCUCCGGUUGCUGAAGGCGTT (SEQ ID NO: 185) | 1.69 |
| RAG9-615 | GGAGCTGTGATCCTAGCTT (SEQ ID NO: 323) | GGAGCUGUGAUCCUAGCUUTT (SEQ ID NO: 49) | AAGCUAGGAUCACAGCUCCTT (SEQ ID NO: 186) | 1.68 |
| RAG9-412 | GAGTCGAATTACAGCCCTG (SEQ ID NO: 324) | GAGUCGAAUUACAGCCCUGTT (SEQ ID NO: 50) | CAGGGCUGUAAUUCGACUCTT (SEQ ID NO: 187) | 1.67 |
| RAG9-162 | AAGGGGCGCAGCGCCTTCA (SEQ ID NO: 325) | AAGGGGCGCAGCGCCUUCATT (SEQ ID NO: 51) | UGAAGGCGCUGCGCCCCUUTT (SEQ ID NO: 188) | 1.66 |
| RAG9-417 | CACCGGAGTCGAATTACAG (SEQ ID NO: 326) | CACCGGAGUCGAAUUACAGTT (SEQ ID NO: 52) | CUGUAAUUCGACUCCGGUGTT (SEQ ID NO: 189) | 1.64 |
| RAG9-324 | TCCTCTGCACACAAGAACT (SEQ ID NO: 327) | UCCUCUGCACACAAGAACUTT (SEQ ID NO: 53) | AGUUCUUGUGUGCAGAGGATT (SEQ ID NO: 190) | 1.61 |
| RAG9-455 | CACCGCTCCCGCACCCCAG (SEQ ID NO: 328) | CACCGCUCCCGCACCCCAGTT (SEQ ID NO: 54) | CUGGGGUGCGGGAGCGGUGTT (SEQ ID NO: 191) | 1.61 |
| RAG9-325 | GTCCTCTGCACACAAGAAC (SEQ ID NO: 329) | GUCCUCUGCACACAAGAACTT (SEQ ID NO: 55) | GUUCUUGUGUGCAGAGGACTT (SEQ ID NO: 192) | 1.61 |
| RAG9-501 | ACATCTGACCCGAGTCAGC (SEQ ID NO: 330) | ACAUCUGACCCGAGUCAGCTT (SEQ ID NO: 56) | GCUGACUCGGGUCAGAUGUTT (SEQ ID NO: 193) | 1.61 |
| RAG9-577 | CGGCCTTCTCTCTGCTTTG (SEQ ID NO: 331) | CGGCCUUCUCUCUGCUUUGTT (SEQ ID NO: 57) | CAAAGCAGAGAGAAGGCCGTT (SEQ ID NO: 194) | 1.60 |
| RAG9-191 | GGAGACTGAGTGGCGCGTG (SEQ ID NO: 332) | GGAGACUGAGUGGCGCGUGTT (SEQ ID NO: 58) | CACGCGCCACUCAGUCUCCTT (SEQ ID NO: 195) | 1.57 |
| RAG9-148 | CTTCAGCAACCGGAGAAGC (SEQ ID NO: 333) | CUUCAGCAACCGGAGAAGCTT (SEQ ID NO: 59) | GCUUCUCCGGUUGCUGAAGTT (SEQ ID NO: 196) | 1.56 |
| RAG9-528 | ATCGCCCAGAGCCGCCCGC (SEQ ID NO: 334) | AUCGCCCAGAGCCGCCCGCTT (SEQ ID NO: 60) | GCGGGCGGCUCUGGGCGAUTT (SEQ ID NO: 197) | 1.56 |
| RAG9-552 | TTCTGGGCGCCTGGCGGGT (SEQ ID NO: 335) | UUCUGGGCGCCUGGCGGGUTT (SEQ ID NO: 61) | ACCCGCCAGGCGCCCAGAATT (SEQ ID NO: 198) | 1.55 |
| RAG9-279 | TTGGCTGCTCGCACGCGCC (SEQ ID NO: 336) | UUGGCUGCUCGCACGCGCCTT (SEQ ID NO: 62) | GGCGCGUGCGAGCAGCCAATT (SEQ ID NO: 199) | 1.53 |
| RAG9-483 | CTTGGGCACCAGCCGAGAG (SEQ ID NO: 337) | CUUGGGCACCAGCCGAGAGTT (SEQ ID NO: 63) | CUCUCGGCUGGUGCCCAAGTT (SEQ ID NO: 200) | 1.52 |
| RAG9-628 | GGCATCAGAAGTGGGAGCT (SEQ ID NO: 338) | GGCAUCAGAAGUGGGAGCUTT (SEQ ID NO: 64) | AGCUCCCACUUCUGAUGCCTT (SEQ ID NO: 201) | 1.52 |
| RAG9-540 | GGCGGGTCCAGAATCGCCC (SEQ ID NO: 339) | GGCGGGUCCAGAAUCGCCCTT (SEQ ID NO: 65) | GGGCGAUUCUGGACCCGCCTT (SEQ ID NO: 202) | 1.50 |
| RAG9-626 | CATCAGAAGTGGGAGCTGT (SEQ ID NO: 340) | CAUCAGAAGUGGGAGCUGUTT (SEQ ID NO: 66) | ACAGCUCCCACUUCUGAUGTT (SEQ ID NO: 203) | 1.50 |
| RAG9-515 | GCCCGCGGTCGTGCACATC (SEQ ID NO: 341) | GCCCGCGGUCGUGCACAUCTT (SEQ ID NO: 67) | GAUGUGCACGACCGCGGGCTT (SEQ ID NO: 204) | 1.49 |
| RAG9-404 | TTACAGCCCTGCAATTAAC (SEQ ID NO: 342) | UUACAGCCCUGCAAUUAACTT (SEQ ID NO: 68) | GUUAAUUGCAGGGCUGUAATT (SEQ ID NO: 205) | 1.49 |
| RAG9-55 | TTGCAGAAGAGACCCGGAA (SEQ ID NO: 343) | UUGCAGAAGAGACCCGGAATT (SEQ ID NO: 69) | UUCCGGGUCUCUUCUGCAATT (SEQ ID NO: 206) | 1.49 |
| RAG9-161 | AGGGGCGCAGCGCCTTCAG (SEQ ID NO: 344) | AGGGGCGCAGCGCCUUCAGTT (SEQ ID NO: 70) | CUGAAGGCGCUGCGCCCCUTT (SEQ ID NO: 207) | 1.48 |
| RAG9-322 | CTCTGCACACAAGAACTTT (SEQ ID NO: 345) | CUCUGCACACAAGAACUUUTT (SEQ ID NO: 71) | AAAGUUCUUGUGUGCAGAGTT (SEQ ID NO: 208) | 1.47 |
| RAG9-106 | TGATCTCCGAGTGGGTGGG (SEQ ID NO: 346) | UGAUCUCCGAGUGGGUGGGTT (SEQ ID NO: 72) | CCCACCCACUCGGAGAUCATT (SEQ ID NO: 209) | 1.46 |
| RAG9-56 | GTTGCAGAAGAGACCCGGA (SEQ ID NO: 347) | GUUGCAGAAGAGACCCGGATT (SEQ ID NO: 73) | UCCGGGUCUCUUCUGCAACTT (SEQ ID NO: 210) | 1.46 |
| RAG9-320 | CTGCACACAAGAACTTTTC (SEQ ID NO: 348) | CUGCACACAAGAACUUUUCTT (SEQ ID NO: 74) | GAAAAGUUCUUGUGUGCAGTT (SEQ ID NO: 211) | 1.44 |
| RAG9-491 | CGAGTCAGCTTGGGCACCA (SEQ ID NO: 349) | CGAGUCAGCUUGGGCACCATT (SEQ ID NO: 75) | UGGUGCCCAAGCUGACUCGTT (SEQ ID NO: 212) | 1.43 |
| RAG9-488 | GTCAGCTTGGGCACCAGCC (SEQ ID NO: 350) | GUCAGCUUGGGCACCAGCCTT (SEQ ID NO: 76) | GGCUGGUGCCCAAGCUGACTT (SEQ ID NO: 213) | 1.43 |
| RAG9-413 | GGAGTCGAATTACAGCCCT (SEQ ID NO: 351) | GGAGUCGAAUUACAGCCCUTT (SEQ ID NO: 77) | AGGGCUGUAAUUCGACUCCTT (SEQ ID NO: 214) | 1.42 |
| RAG9-512 | CGCGGTCGTGCACATCTGA (SEQ ID NO: 352) | CGCGGUCGUGCACAUCUGATT (SEQ ID NO: 78) | UCAGAUGUGCACGACCGCGTT (SEQ ID NO: 215) | 1.42 |
| RAG9-113 | TGGTGTGTGATCTCCGAGT (SEQ ID NO: 353) | UGGUGUGUGAUCUCCGAGUTT (SEQ ID NO: 79) | ACUCGGAGAUCACACACCATT (SEQ ID NO: 216) | 1.42 |
| RAG9-274 | TGCTCGCACGCGCCTGCCC (SEQ ID NO: 354) | UGCUCGCACGCGCCUGCCCTT (SEQ ID NO: 80) | GGGCAGGCGCGUGCGAGCATT (SEQ ID NO: 217) | 1.41 |
| RAG9-634 | TGCTTGGGCATCAGAAGTG (SEQ ID NO: 355) | UGCUUGGGCAUCAGAAGUGTT (SEQ ID NO: 81) | CACUUCUGAUGCCCAAGCATT (SEQ ID NO: 218) | 1.39 |
| RAG9-558 | AGTGGCTTCTGGGCGCCTG (SEQ ID NO: 356) | AGUGGCUUCUGGGCGCCUGTT (SEQ ID NO: 82) | CAGGCGCCCAGAAGCCACUTT (SEQ ID NO: 219) | 1.39 |
| RAG9-326 | AGTCCTCTGCACACAAGAA (SEQ ID NO: 357) | AGUCCUCUGCACACAAGAATT (SEQ ID NO: 83) | UUCUUGUGUGCAGAGGACUTT (SEQ ID NO: 220) | 1.38 |
| RAG9-110 | TGTGTGATCTCCGAGTGGG (SEQ ID NO: 358) | UGUGUGAUCUCCGAGUGGGTT (SEQ ID NO: 84) | CCCACUCGGAGAUCACACATT (SEQ ID NO: 221) | 1.37 |
| RAG9-340 | TGAGCAGGCTTGGGAGTCC (SEQ ID NO: 359) | UGAGCAGGCUUGGGAGUCCTT (SEQ ID NO: 85) | GGACUCCCAAGCCUGCUCATT (SEQ ID NO: 222) | 1.37 |
| RAG9-506 | CGTGCACATCTGACCCGAG (SEQ ID NO: 360) | CGUGCACAUCUGACCCGAGTT (SEQ ID NO: 86) | CUCGGGUCAGAUGUGCACGTT (SEQ ID NO: 223) | 1.36 |
| RAG9-507 | TCGTGCACATCTGACCCGA (SEQ ID NO: 361) | UCGUGCACAUCUGACCCGATT (SEQ ID NO: 87) | UCGGGUCAGAUGUGCACGATT (SEQ ID NO: 224) | 1.36 |
| RAG9-1407 | AGCTTCTGATTGGCTCTTT (SEQ ID NO: 362) | AGCUUCUGAUUGGCUCUUUTT (SEQ ID NO: 88) | AAAGAGCCAAUCAGAAGCUTT (SEQ ID NO: 225) | 1.35 |
| RAG9-508 | GTCGTGCACATCTGACCCG (SEQ ID NO: 363) | GUCGUGCACAUCUGACCCGTT (SEQ ID NO: 89) | CGGGUCAGAUGUGCACGACTT (SEQ ID NO: 226) | 1.35 |
| RAG9-588 | CACAGGGTAGGCGGCCTTC (SEQ ID NO: 364) | CACAGGGUAGGCGGCCUUCTT (SEQ ID NO: 90) | GAAGGCCGCCUACCCUGUGTT (SEQ ID NO: 227) | 1.35 |
| RAG9-114 | CTGGTGTGTGATCTCCGAG (SEQ ID NO: 365) | CUGGUGUGUGAUCUCCGAGTT (SEQ ID NO: 91) | CUCGGAGAUCACACACCAGTT (SEQ ID NO: 228) | 1.34 |
| RAG9-456 | GCACCGCTCCCGCACCCCA (SEQ ID NO: 366) | GCACCGCUCCCGCACCCCATT (SEQ ID NO: 92) | UGGGGUGCGGGAGCGGUGCTT (SEQ ID NO: 229) | 1.34 |
| RAG9-312 | AAGAACTTTTCTCGGGGTG (SEQ ID NO: 367) | AAGAACUUUUCUCGGGGUGTT (SEQ ID NO: 93) | CACCCCGAGAAAAGUUCUUTT (SEQ ID NO: 230) | 1.34 |
| RAG9-315 | CACAAGAACTTTTCTCGGG (SEQ ID NO: 368) | CACAAGAACUUUUCUCGGGTT (SEQ ID NO: 94) | CCCGAGAAAAGUUCUUGUGTT (SEQ ID NO: 231) | 1.33 |
| RAG9-575 | GCCTTCTCTCTGCTTTGAG (SEQ ID NO: 369) | GCCUUCUCUCUGCUUUGAGTT (SEQ ID NO: 95) | CUCAAAGCAGAGAGAAGGCTT (SEQ ID NO: 232) | 1.32 |
| RAG9-467 | GAGCCGGCTCCGCACCGCT (SEQ ID NO: 370) | GAGCCGGCUCCGCACCGCUTT (SEQ ID NO: 96) | AGCGGUGCGGAGCCGGCUCTT (SEQ ID NO: 233) | 1.32 |
| RAG9-231 | ACGCGACCGGCGCGAGGAG (SEQ ID NO: 371) | ACGCGACCGGCGCGAGGAGTT (SEQ ID NO: 97) | CUCCUCGCGCCGGUCGCGUTT (SEQ ID NO: 234) | 1.31 |
| RAG9-637 | AAATGCTTGGGCATCAGAA (SEQ ID NO: 372) | AAAUGCUUGGGCAUCAGAATT (SEQ ID NO: 98) | UUCUGAUGCCCAAGCAUUUTT (SEQ ID NO: 235) | 1.31 |
| RAG9-633 | GCTTGGGCATCAGAAGTGG (SEQ ID NO: 373) | GCUUGGGCAUCAGAAGUGGTT (SEQ ID NO: 99) | CCACUUCUGAUGCCCAAGCTT (SEQ ID NO: 236) | 1.30 |
| RAG9-519 | AGCCGCCCGCGGTCGTGCA (SEQ ID NO: 374) | AGCCGCCCGCGGUCGUGCATT (SEQ ID NO: 100) | UGCACGACCGCGGGCGGCUTT (SEQ ID NO: 237) | 1.30 |
| RAG9-509 | GGTCGTGCACATCTGACCC (SEQ ID NO: 375) | GGUCGUGCACAUCUGACCCTT (SEQ ID NO: 101) | GGGUCAGAUGUGCACGACCTT (SEQ ID NO: 238) | 1.29 |
| RAG9-146 | TCAGCAACCGGAGAAGCAT (SEQ ID NO: 376) | UCAGCAACCGGAGAAGCAUTT (SEQ ID NO: 102) | AUGCUUCUCCGGUUGCUGATT (SEQ ID NO: 239) | 1.29 |
| RAG9-531 | AGAATCGCCCAGAGCCGCC (SEQ ID NO: 377) | AGAAUCGCCCAGAGCCGCCTT (SEQ ID NO: 103) | GGCGGCUCUGGGCGAUUCUTT (SEQ ID NO: 240) | 1.29 |
| RAG9-614 | GAGCTGTGATCCTAGCTTG (SEQ ID NO: 378) | GAGCUGUGAUCCUAGCUUGTT (SEQ ID NO: 104) | CAAGCUAGGAUCACAGCUCTT (SEQ ID NO: 241) | 1.29 |
| RAG9-138 | CGGAGAAGCATAGTTGCAC (SEQ ID NO: 379) | CGGAGAAGCAUAGUUGCACTT (SEQ ID NO: 105) | GUGCAACUAUGCUUCUCCGTT (SEQ ID NO: 242) | 1.29 |
| RAG9-129 | ATAGTTGCACGCGACCTGG (SEQ ID NO: 380) | AUAGUUGCACGCGACCUGGTT (SEQ ID NO: 106) | CCAGGUCGCGUGCAACUAUTT (SEQ ID NO: 243) | 1.28 |
| RAG9-308 | ACTTTTCTCGGGGTGTAAA (SEQ ID NO: 381) | ACUUUUCUCGGGGUGUAAATT (SEQ ID NO: 107) | UUUACACCCCGAGAAAAGUTT (SEQ ID NO: 244) | 1.28 |
| RAG9-553 | CTTCTGGGCGCCTGGCGGG (SEQ ID NO: 382) | CUUCUGGGCGCCUGGCGGGTT (SEQ ID NO: 108) | CCCGCCAGGCGCCCAGAAGTT (SEQ ID NO: 245) | 1.27 |
| RAG9-439 | CAGCCGCCGGGGTGGTGAC (SEQ ID NO: 383) | CAGCCGCCGGGGUGGUGACTT (SEQ ID NO: 109) | GUCACCACCCCGGCGGCUGTT (SEQ ID NO: 246) | 1.27 |
| RAG9-613 | AGCTGTGATCCTAGCTTGG (SEQ ID NO: 384) | AGCUGUGAUCCUAGCUUGGTT (SEQ ID NO: 110) | CCAAGCUAGGAUCACAGCUTT (SEQ ID NO: 247) | 1.26 |
| RAG9-119 | GCGACCTGGTGTGTGATCT (SEQ ID NO: 385) | GCGACCUGGUGUGUGAUCUTT (SEQ ID NO: 111) | AGAUCACACACCAGGUCGCTT (SEQ ID NO: 248) | 1.25 |
| RAG9-590 | AGCACAGGGTAGGCGGCCT (SEQ ID NO: 386) | AGCACAGGGUAGGCGGCCUTT (SEQ ID NO: 112) | AGGCCGCCUACCCUGUGCUTT (SEQ ID NO: 249) | 1.25 |
| RAG9-546 | GCGCCTGGCGGGTCCAGAA (SEQ ID NO: 387) | GCGCCUGGCGGGUCCAGAATT (SEQ ID NO: 113) | UUCUGGACCCGCCAGGCGCTT (SEQ ID NO: 250) | 1.25 |
| RAG9-627 | GCATCAGAAGTGGGAGCTG (SEQ ID NO: 388) | GCAUCAGAAGUGGGAGCUGTT (SEQ ID NO: 114) | CAGCUCCCACUUCUGAUGCTT (SEQ ID NO: 251) | 1.24 |
| RAG9-530 | GAATCGCCCAGAGCCGCCC (SEQ ID NO: 389) | GAAUCGCCCAGAGCCGCCCTT (SEQ ID NO: 115) | GGGCGGCUCUGGGCGAUUCTT (SEQ ID NO: 252) | 1.24 |
| RAG9-159 | GGGCGCAGCGCCTTCAGCA (SEQ ID NO: 390) | GGGCGCAGCGCCUUCAGCATT (SEQ ID NO: 116) | UGCUGAAGGCGCUGCGCCCTT (SEQ ID NO: 253) | 1.24 |
| RAG9-403 | TACAGCCCTGCAATTAACA (SEQ ID NO: 391) | UACAGCCCUGCAAUUAACATT (SEQ ID NO: 117) | UGUUAAUUGCAGGGCUGUATT (SEQ ID NO: 254) | 1.23 |
| RAG9-388 | AACATATGAATCTGACGAA (SEQ ID NO: 392) | AACAUAUGAAUCUGACGAATT (SEQ ID NO: 118) | UUCGUCAGAUUCAUAUGUUTT (SEQ ID NO: 255) | 1.22 |
| RAG9-454 | ACCGCTCCCGCACCCCAGC (SEQ ID NO: 393) | ACCGCUCCCGCACCCCAGCTT (SEQ ID NO: 119) | GCUGGGGUGCGGGAGCGGUTT (SEQ ID NO: 256) | 1.22 |
| RAG9-189 | AGACTGAGTGGCGCGTGCC (SEQ ID NO: 394) | AGACUGAGUGGCGCGUGCCTT (SEQ ID NO: 120) | GGCACGCGCCACUCAGUCUTT (SEQ ID NO: 257) | 1.21 |
| RAG9-131 | GCATAGTTGCACGCGACCT (SEQ ID NO: 395) | GCAUAGUUGCACGCGACCUTT (SEQ ID NO: 121) | AGGUCGCGUGCAACUAUGCTT (SEQ ID NO: 258) | 1.20 |
| RAG9-504 | TGCACATCTGACCCGAGTC (SEQ ID NO: 396) | UGCACAUCUGACCCGAGUCTT (SEQ ID NO: 122) | GACUCGGGUCAGAUGUGCATT (SEQ ID NO: 259) | 1.19 |
| RAG9-529 | AATCGCCCAGAGCCGCCCG (SEQ ID NO: 397) | AAUCGCCCAGAGCCGCCCGTT (SEQ ID NO: 123) | CGGGCGGCUCUGGGCGAUUTT (SEQ ID NO: 260) | 1.19 |
| RAG9-493 | CCCGAGTCAGCTTGGGCAC (SEQ ID NO: 398) | CCCGAGUCAGCUUGGGCACTT (SEQ ID NO: 124) | GUGCCCAAGCUGACUCGGGTT (SEQ ID NO: 261) | 1.19 |
| RAG9-481 | TGGGCACCAGCCGAGAGCC (SEQ ID NO: 399) | UGGGCACCAGCCGAGAGCCTT (SEQ ID NO: 125) | GGCUCUCGGCUGGUGCCCATT (SEQ ID NO: 262) | 1.18 |
| RAG9-307 | CTTTTCTCGGGGTGTAAAA (SEQ ID NO: 400) | CUUUUCUCGGGGUGUAAAATT (SEQ ID NO: 126) | UUUUACACCCCGAGAAAAGTT (SEQ ID NO: 263) | 1.16 |
| RAG9-564 | GCTTTGAGTGGCTTCTGGG (SEQ ID NO: 401) | GCUUUGAGUGGCUUCUGGGTT (SEQ ID NO: 127) | CCCAGAAGCCACUCAAAGCTT (SEQ ID NO: 264) | 1.16 |
| RAG9-410 | GTCGAATTACAGCCCTGCA (SEQ ID NO: 402) | GUCGAAUUACAGCCCUGCATT (SEQ ID NO: 128) | UGCAGGGCUGUAAUUCGACTT (SEQ ID NO: 265) | 1.15 |
| RAG9-511 | GCGGTCGTGCACATCTGAC (SEQ ID NO: 403) | GCGGUCGUGCACAUCUGACTT (SEQ ID NO: 129) | GUCAGAUGUGCACGACCGCTT (SEQ ID NO: 266) | 1.15 |
| RAG9-406 | AATTACAGCCCTGCAATTA (SEQ ID NO: 404) | AAUUACAGCCCUGCAAUUATT (SEQ ID NO: 130) | UAAUUGCAGGGCUGUAAUUTT (SEQ ID NO: 267) | 1.15 |
| RAG9-58 | ACGTTGCAGAAGAGACCCG (SEQ ID NO: 405) | ACGUUGCAGAAGAGACCCGTT (SEQ ID NO: 131) | CGGGUCUCUUCUGCAACGUTT (SEQ ID NO: 268) | 1.15 |
| RAG9-547 | GGCGCCTGGCGGGTCCAGA (SEQ ID NO: 406) | GGCGCCUGGCGGGUCCAGATT (SEQ ID NO: 132) | UCUGGACCCGCCAGGCGCCTT (SEQ ID NO: 269) | 1.15 |
| RAG9-123 | GCACGCGACCTGGTGTGTG (SEQ ID NO: 407) | GCACGCGACCUGGUGUGUGTT (SEQ ID NO: 133) | CACACACCAGGUCGCGUGCTT (SEQ ID NO: 270) | 1.14 |
| RAG9-124 | TGCACGCGACCTGGTGTGT (SEQ ID NO: 408) | UGCACGCGACCUGGUGUGUTT (SEQ ID NO: 134) | ACACACCAGGUCGCGUGCATT (SEQ ID NO: 271) | 1.14 |
| RAG9-436 | CCGCCGGGGTGGTGACACA (SEQ ID NO: 409) | CCGCCGGGGUGGUGACACATT (SEQ ID NO: 135) | UGUGUCACCACCCCGGCGGTT (SEQ ID NO: 272) | 1.14 |
| RAG9-286 | TCTTTGATTGGCTGCTCGC (SEQ ID NO: 410) | UCUUUGAUUGGCUGCUCGCTT (SEQ ID NO: 136) | GCGAGCAGCCAAUCAAAGATT (SEQ ID NO: 273) | 1.13 |
| RAG9-420 | ACACACCGGAGTCGAATTA (SEQ ID NO: 411) | ACACACCGGAGUCGAAUUATT (SEQ ID NO: 137) | UAAUUCGACUCCGGUGUGUTT (SEQ ID NO: 274) | 1.13 |
| RAG9-636 | AATGCTTGGGCATCAGAAG (SEQ ID NO: 412) | AAUGCUUGGGCAUCAGAAGTT (SEQ ID NO: 138) | CUUCUGAUGCCCAAGCAUUTT (SEQ ID NO: 275) | 1.13 |
| RAG9-289 | AACTCTTTGATTGGCTGCT (SEQ ID NO: 413) | AACUCUUUGAUUGGCUGCUTT (SEQ ID NO: 139) | AGCAGCCAAUCAAAGAGUUTT (SEQ ID NO: 276) | 1.13 |
| RAG9-537 | GGGTCCAGAATCGCCCAGA (SEQ ID NO: 414) | GGGUCCAGAAUCGCCCAGATT (SEQ ID NO: 140) | UCUGGGCGAUUCUGGACCCTT (SEQ ID NO: 277) | 1.12 |
| RAG9-419 | CACACCGGAGTCGAATTAC (SEQ ID NO: 415) | CACACCGGAGUCGAAUUACTT (SEQ ID NO: 141) | GUAAUUCGACUCCGGUGUGTT (SEQ ID NO: 278) | 1.12 |
| RAG9-327 | GAGTCCTCTGCACACAAGA (SEQ ID NO: 416) | GAGUCCUCUGCACACAAGATT (SEQ ID NO: 142) | UCUUGUGUGCAGAGGACUCTT (SEQ ID NO: 279) | 1.10 |

It can be clearly seen from the arrangement of activities of the 403 saRNAs according to their positions within the *ATOH1* promoter region that the functional saRNAs are gathered together, that is, in certain promoter regions, the activating saRNAs gathered in specific "hotspot" regions (FIG. 2). As shown in FIG. 2, 4 hotspot regions respectively appeared in region (H1) from -578 to -386, region (H2) from -339 to -291, region (H3) from -162 to -86 and region (H4) from -55 to -34 of the promoter, respectively, showing high gathering of the activating saRNAs, and the proportion of functional saRNAs was 41.0%, 51.6%, 45.6%, and 100%, respectively. These results indicate that the functional saRNAs were not randomly distributed witin the promoter, but existed in the specific hotspot regions.

The sequence of the hotspot H1 (5' to 3': -578 to -386) corresponds to SEQ ID NO: 2 in the sequence listing:

gcggccttctctctgctttgagtggcttctgggcgcctggcgggtccagaatcgcccagagccgcccgcggtcgtgcacatctgacccgagtca
gcttgggcaccagccgagagccggctccgcaccgctcccgcaccccagccgccggggtggtgacacacaccggagtcgaattacagccct
gcaattaa

The sequence of the hotspot H2 (5' to 3': -339 to -291) corresponds to SEQ ID NO: 3 in the sequence listing:
tgcacacaagaacttttctcggggtgtaa

The sequence of the hotspot H3 (5' to 3': -162 to -86) corresponds to SEQ ID NO: 4 in the sequence listing:
aaggggcgcagcgccttcagcaaccggagaagcatagttgcacgcgacctggtgtgtgatctccgagtgggtgggg

The sequence of the hotspot H4 (5' to 3': -55 to -34) corresponds to SEQ ID NO: 5 in the sequence listing:
ttgcagaagagacccggaaagg

### Example 3: Validation of High-throughput Screening Results

Normal Human Epidermal Keratinocytes (NHEK) cells and human renal embryonic cells (HEK293T) were seeded into a 6-well plate with 2×10⁵ to 3×10⁵ cells/well and were reverse transfected with oligonucleotide duplexes. Total cellular RNAs were extracted using an RNeasy Plus Mini kit (Qiagen). The resulting RNA (1 µg) was reverse transcribed into cDNA using a PrimeScript RT kit containing gDNA Eraser (Takara, Shlga, Japan). The resulting cDNA was amplified by qPCR using an ABI 7500 fast real-time PCR system (Applied Biosystems) and SYBR Premix Ex Taq II reagent (Takara, Shlga, Japan). The reaction conditions were: 95°C for 3 s, 60°C for 30 s, and 40 cycles. HPRT1 was used as internal control and RAG9-871i as inhibitory dsRNA control. The phase expression value of the *ATOH1* gene was calculated by a method of 2^{-ΔΔ}*^{CT}.* All the primer sequences used are listed in Table 3.

In order to calculate the expression value (*Eᵣₑₗ*) of *ATOH1* (target gene) of an saRNAtransfected sample relative to control treatment (mock), the Ct values of the target gene and the internal control genes were substituted into Formula 1 for calculation.

*E*_{*rel* =2}^{(*CtTm-CtTs*)} /₂^{(*CtRm-CtRs*)} (Formula 1)

wherein CtTm was the Ct value of the target gene from Mock sample; CtTs was the Ct value of the target gene from the saRNA-treated sample; CtRm was the Ct value of the internal control gene from mock-treated sample; CtRs was the Ct value of the internal control gene from the saRNA-treated sample.

**Table 3. Primer sequences for two-step qRT-PCR analysis**

| **Primer** | **Sequence No.** | **Sequence (5'-3')** |
|---|---|---|
| ATOH1 F1 | SEQ ID NO: 417 | CCCTTCCAGCAAACAGGTGA |
| ATOH1 R1 | SEQ ID NO: 418 | GAACGACGGGATAACATTGCG |
| HPRT1 F | SEQ ID NO: 419 | ATGGACAGGACTGAACGTCTT |
| HPRT1 R | SEQ ID NO: 420 | TCCAGCAGGTCAGCAAAGAA |

As shown in **FIG. 3** and **FIG. 4****,** randomly selected functional saRNAs were verified in HEK293T cells and NHEK cells, and both showed up-regulation of mRNA expression of ATOH1.

**Table 4. Double-stranded RNA sequences as study controls**

| **Double-stranded RNA** | **Sequence No.** | **Sequence (5'-3')** |
|---|---|---|
| dsCon2-sense strand | SEQ ID NO: 421 | ACUACUGAGUGACAGUAGATT |
| dsCon2-antisense strand | SEQ ID NO: 422 | UCUACUGUCACUCAGUAGUTT |
| RAG9-871i-sense strand | SEQ ID NO: 423 | GCUCUGCACUUCUCGACUUTT |
| RAG9-871i-antisense strand | SEQ ID NO: 424 | AAGUCGAGAAGUGCAGAGCTT |

### Example 4. saRNAs Promoting ATOH1 Protein Expression

HEK293T cells were plated into a 96-well plate at a concentration of 3×10³ to 5×10³ cells/well, cultured overnight, and transfected with 7 randomly selected functional saRNAs. Seventy two hours after transfection, cells were collected and lysed using a cell lysis buffer (1×RIPA buffer, CST) which contained a protease inhibitor. Proteins were quantified by BCA method (Thermo) and separated by polyacrylamide gel electrophoresis and conversion to a 0.45 µm PVDF membrane. Primary Rabbit monoclonal anti-ATOH1 (Abcam) and α/β-tubulin antibodies (Cell Signaling Technology) were used to detect blots and a secondary anti-rabbit IgG, HRP-linked antibody (Cell Signaling Technology) was used. Signals were detected using an Image Lab (BIO-RAD, Chemistry Doctm MP imaging system).

As shown in **FIG. 5****,** the 7 randomly selected activating saRNAs were capable of promoting or increasing the expression of ATOH1 protein.

Based on these results, a plurality of saRNAs capable of activating the expression of *ATOH1* gene were found through high-throughput screening of saRNAs targeting *ATOH1* gene promoter. The saRNAs can up-regulate the expression of the *ATOH1* gene on mRNA and at the protein expression level, and can be used to treat a disease or symptom caused by a decrease or insufficient expression of the ATOH1 protein, or used in the preparation of a method or drug for treating the disease or symptom.

### References

1. Aragaki, M., K. Tsuchiya, R. Okamoto, S. Yoshioka, T. Nakamura, N. Sakamoto, T. Kanai, and M. Watanabe. 2008. 'Proteasomal degradation of Atoh1 by aberrant Wnt signaling maintains the undifferentiated state of colon cancer', Biochem Biophys Res Commun, 368: 923-9.
2. Bermingham, N. A., B. A. Hassan, S. D. Price, M. A. Vollrath, N. Ben-Arie, R. A. Eatock, H. J. Bellen, A. Lysakowski, and H. Y. Zoghbi. 1999. 'Math1: an essential gene for the generation of inner ear hair cells', Science, 284: 1837-41.
3. Gubbels, S. P., D. W. Woessner, J. C. Mitchell, A. J. Ricci, and J. V. Brigande. 2008. 'Functional auditory hair cells produced in the mammalian cochlea by in utero gene transfer', Nature, 455: 537-41.
4. Gurtler, N., and A. K. Lalwani. 2002. 'Etiology of syndromic and nonsyndromic sensorineural hearing loss', Otolaryngol Clin North Am, 35: 891-908.
5. Izumikawa, M., R. Minoda, K. Kawamoto, K. A. Abrashkin, D. L. Swiderski, D. F. Dolan, D. E. Brough, and Y. Raphael. 2005. 'Auditory hair cell replacement and hearing improvement by Atoh1 gene therapy in deaf mammals', Nat Med, 11: 271-6.
6. Kawamoto, K., S. Ishimoto, R. Minoda, D. E. Brough, and Y. Raphael. 2003. 'Math1 gene transfer generates new cochlear hair cells in mature guinea pigs in vivo', J Neurosci, 23: 4395-400.
7. Kazanjian A., Shroyer S.F. 2011. NOTCH Signaling and ATOH1 in Colorectal Cancers. Curr Colorectal Cancer Rep Jun; 7(2):121-127.
8. Leow, C. C., P. Polakis, and W. Q. Gao. 2005. 'A role for Hath1, a bHLH transcription factor, in colon adenocarcinoma', Ann N Y Acad Sci, 1059: 174-83.
9. Liberman, M. C. 1990. 'Quantitative assessment of inner ear pathology following ototoxic drugs or acoustic trauma', Toxicol Pathol, 18: 138-48.
10. Richardson, R. T., and P. J. Atkinson. 2015. 'Atoh1 gene therapy in the cochlea for hair cell regeneration', Expert Opin Biol Ther, 15: 417-30.
11. Zheng, J. L., and W. Q. Gao. 2000. 'Overexpression of Math1 induces robust production of extra hair cells in postnatal rat inner ears', Nat Neurosci, 3: 580-6.

## Claims

1. A small activating nucleic acid molecule, comprising a first nucleic acid strand and a second nucleic acid strand, wherein the first nucleic acid strand has at least 75% homology or complementarity to a continuous sequence of 16 to 35 nucleotides in length in positions -578 to -386 (SEQ ID NO: 2), positions -339 to -291 (SEQ ID NO: 3), positions -162 to -86 (SEQ ID NO: 4) or positions -55 to -34 (SEQ ID NO: 5) upstream of the transcription start site in a promoter region of an *ATOH1* gene, wherein the first nucleic acid strand and the second nucleic acid strand complementarily form a double-stranded nucleic acid structure capable of activating/up-regulating the expression of *ATOH1* gene in a cell.

2. The small activating nucleic acid molecule of claim 1, wherein the first nucleic acid strand and the second nucleic acid strand are present on two different nucleic acid strands.

3. The small activating nucleic acid molecule of claim 1, wherein the first nucleic acid strand and the second nucleic acid strand are present on the same nucleic acid strand, and preferably, the small activating nucleic acid molecule is a hairpin single-stranded nucleic acid molecule, wherein the first nucleic acid strand and the second nucleic acid strand comprise complementary regions forming the double-stranded nucleic acid structure.

4. The small activating nucleic acid molecule of claim 2, wherein at least one strand of the small activating nucleic acid molecule has an overhang of 0 to 6 nucleotides in length at the 3' terminus of the strand.

5. The small activating nucleic acid molecule of claim 4, wherein both strands of the small activating nucleic acid molecule have overhangs of 0 to 6 nucleotides in length at the 3' terminus of each strand, preferably the overhangs are of 2 or 3 nucleotides in length.

6. The small activating nucleic acid molecule of any one of claims 1 to 5, wherein the first nucleic acid strand and the second nucleic acid strand independently have 16 to 35 nucleotides in length.

7. The small activating nucleic acid molecule of any one of claims 1 to 6, wherein one strand of the small activating nucleic acid molecule comprises a nucleic acid sequence having at least 75% homology or complementarity to a nucleotide sequence selected from the group consisting of SEQ ID NOs: 280-416, or consists of a nucleic acid sequence having at least 75% homology or complementarity to a nucleotide sequence selected from the group consisting of SEQ ID NOs: 280-416.

8. The small activating nucleic acid molecule of any one of claims 1 to 7, wherein the first nucleic acid strand has at least 75% homology to a nucleotide sequence selected from the group consisting of SEQ ID NOs: 6-142, and the antisense nucleic acid fragment has at least 75% homology to any nucleotide sequence selected from the group consisting of SEQ ID NOs: 143-279.

9. The small activating nucleic acid molecule of claims 1 to 8, wherein the first nucleic acid strand comprises any nucleotide sequence selected from the group consisting of SEQ ID NOs: 6-142, or consists of any nucleotide sequence selected from SEQ ID NOs: 6-142; the second nucleic acid strand comprises any nucleotide sequence selected from the group consisting of SEQ ID NOs: 143-279, or consists of any nucleotide sequence selected from SEQ ID NOs: 143-279.

10. The small activating nucleic acid molecule of any one of claims 1 to 9, wherein the small activating nucleic acid molecule comprises at least one modification, preferably the modification is a chemical modification.

11. The small activating nucleic acid molecule of claim 10, wherein the chemical modification comprises or is selected from one or more modifications selected from the group consisting of:
(1) a modification of a phosphodiester bond connecting nucleotides in the nucleotide sequence of the small activating nucleic acid molecule;
(2) a modification of 2'-OH of a ribose in the nucleotide sequence of the small activating nucleic acid molecule; and
(3) a modification of a base in the nucleotide sequence of the small activating nucleic acid molecule; and
(4) at least one nucleotide in the nucleotide sequence of the small activating nucleic acid molecule being a locked nucleic acid.

12. The small activating nucleic acid molecule of claim 10, wherein the chemical modification comprises or is selected from one or more modifications selected from the group consisting of: 2'-fluoro modification, 2'-oxymethyl modification, 2'-oxyethylidene methoxy modification, 2,4'-dinitrophenol modification, locked nucleic acid (LNA), 2'-amino modification, 2'-deoxy modification, 5'-bromouracil modification, 5'-iodouracil modification, N-methyluracil modification, 2,6-diaminopurine modification, phosphorothioate modification, and boranophosphate modification.

13. The small activating nucleic acid molecule of any one of claims 1-12, wherein the small activating nucleic acid molecule activates or upregulates the expression of the *ATOH1* gene by at least 10%.

14. A nucleic acid encoding the small activating nucleic acid molecule of any one of claims 1 to 9.

15. The nucleic acid of claim 14, wherein the nucleic acid is a DNA molecule.

16. A cell comprising the small activating nucleic acid molecule of any one of claims 1 to 13 or the nucleic acid of claim 14 or 15.

17. A composition comprising the small activating nucleic acid molecule of any one of claims 1 to 13, the nucleic acid of claim 14 or 15, or the cell of claim 16, and optionally, a pharmaceutically acceptable carrier.

18. The composition of claim 17, wherein the pharmaceutically acceptable carrier is selected from the group consisting of: a liposome, a high-molecular polymer, and a polypeptide.

19. A kit comprising the small activating nucleic acid molecule of any one of claims 1 to 13, the nucleic acid of claim 14 or 15, the cell of claim 16, or the composition of any one of claims 17 to 18.

20. A use of the small activating nucleic acid molecule of any one of claims 1 to 13, the nucleic acid of claim 14 or 15 or the composition of any one of claims 17 to 18 in preparing a formulation for activating or upregulating the expression of the *ATOH1* gene in a cell.

21. The use of claim 20, wherein the small activating nucleic acid molecule is directly introduced into the cell.

22. The use of claim 20, wherein the small activating nucleic acid molecule is produced in the cell after a nucleotide sequence encoding the small activating nucleic acid molecule is introduced into the cell.

23. The use of any one of claims 20 to 22, wherein the cell is a mammalian cell.

24. The use of claim 20, wherein the cell is a human cell.

25. The use of claim 24, wherein the cell is present in a human body.

26. The use of claim 25, wherein the human body is a patient suffering from a disease or condition related to insufficient or decreased expression of the ATOH1 protein, and the small activating nucleic acid molecule is administered at a sufficient dose to treat the disease or condition.

27. The use of claim 26, wherein the disease or condition related to insufficient or decreased expression of the ATOH1 protein comprises hearing loss and tumor.

28. An isolated target site of the small *ATOH1* -activating nucleic acid molecule, wherein the target site comprises or is selected from any continuous sequence of 16 to 35 nucleotides in length from a nucleotide sequence selected from the group consisting of: SEQ ID NOs: 2-5.

29. The small activating nucleic acid molecule target site of claim 28, wherein the target site comprises or is selected from any of a nucleotide sequence selected from the group consisting of:
SEQ ID NOs: 280-416.

30. A method for activating or upregulating the expression of an *ATOH1* gene in a cell, comprising administering the small activating nucleic acid molecule of any one of claims 1 to 13, the nucleic acid of claim 14 or 15, or the composition of any one of claims 17 to 18 to the cell.

31. The method of claim 30, wherein the small activating nucleic acid molecule is directly introduced into the cell.

32. The method of claim 30, wherein the small activating nucleic acid molecule is produced in the cell after the nucleic acid encoding the small activating nucleic acid molecule is introduced into the cell.

33. The method of any one of claims 30 to 32, wherein the cell is a mammalian cell.

34. The method of claim 30, wherein the cell is a human cell.

35. The method of claim 34, wherein the cell is present in a human body.

36. The method of claim 35, wherein the human body is a patient suffering from a disease or condition related to insufficient or decreased expression of the ATOH1 protein, and the small activating nucleic acid molecule is administered at a sufficient dose to treat the disease or condition.

37. The method of claim 35, wherein the disease or condition related to insufficient or decreased expression of the ATOH1 protein comprises hearing loss and tumor.

38. A method for treating a disease or condition related to insufficient or decreased expression of the ATOH1 protein in an individual, comprising administering the small activating nucleic acid molecule of any one of claims 1 to 13, the nucleic acid of claim 14 or 15, the cell of claim 16 or the composition of any one of claims 17 to 18 at a curative dose to the individual.

39. The use of claim 38, wherein the disease or condition related to insufficient or decreased expression of the ATOH1 protein comprises hearing loss and tumor.

40. The method of claim 38 or 39, wherein the individual is a mammal.

41. The method of claim 38, wherein the individual is a human.

42. A use of the small activating nucleic acid molecule of any one of claims 1 to 13, the nucleic acid of claim 14 or 15, the cell of claim 16 or the composition of any one of claims 17 to 18 in preparing a drug for treating a disease or condition related to insufficient or decreased expression of the ATOH1 protein.

43. The use of claim 42, wherein the disease or condition related to insufficient or decreased expression of the ATOH1 protein comprises hearing loss and tumor.

44. A use of the small activating nucleic acid molecule of any one of claims 1 to 13, the nucleic acid of claim 14 or 15, the cell of claim 16 or the composition of any one of claims 17 to 18 in preparing a drug for treating a disease caused by insufficient expression of the ATOH1 protein, wherein the disease comprises hearing loss.

45. The use of any one of claims 43 to 44, wherein the individual is a mammal.

46. The use of any one of claims 43 to 44, wherein the individual is a human.
